(19) Europäisches Patentamt
European Patent Office
Office européen des brevets

(11) **EP 4 686 725 A1**

(12) **EUROPEAN PATENT APPLICATION**

(43) Date of publication:
**04.02.2026 Bulletin 2026/06**

(21) Application number: **24192715.1**

(22) Date of filing: **02.08.2024**

(51) International Patent Classification (IPC):
***C07K 7/08*** *(2006.01)* ***G01N 33/68*** *(2006.01)*

(52) Cooperative Patent Classification (CPC):
**C07K 7/08; G01N 33/6896**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Technische Universität München, in Vertretung des Freistaats Bayern**
**80333 München (DE)**

(72) Inventor: **The designation of the inventor has not yet been filed**

(74) Representative: **Engelhard, Markus**
**Boehmert & Boehmert**
**Anwaltspartnerschaft mbB**
**Pettenkoferstrasse 22**
**80336 München (DE)**

# (54) AMYLOID INHIBITORY PEPTIDES

(57) The present invention relates to peptides, in particular of amyloid inhibitory peptides, and to pharmaceutical compositions comprising such peptides, for use in methods of treating or preventing or delaying the onset of synucleinopathies, in particular of Parkinson's disease (PD) or dementia with Lewy bodies, and their comorbidities, in particular PD/type 2 diabetes (T2D) and PD/Alzheimer's disease (AD). Furthermore, the present invention relates to such peptides, in particular such amyloid inhibitory peptides, for use in methods of diagnosing such synucleinopathies and related comorbidities. Furthermore, the present invention also relates to a kit for the in-vitro or in-vivo detection and, optionally, quantification of amyloidogenic polypeptides, amyloid fibrils or amyloid aggregates, and/or for the diagnosis of synucleinopathies and related comorbidities, in particular PD/type 2 diabetes (T2D) and PD/Alzheimer's disease (AD), in a patient.

Figure 1

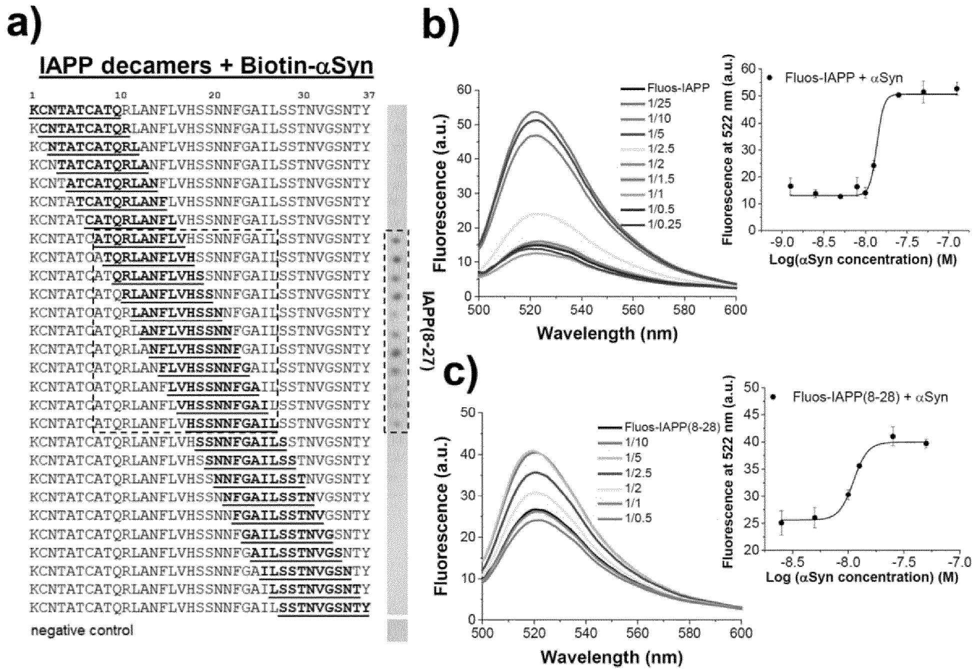


EP 4 686 725 A1

## Description

The present invention relates to peptides, in particular of amyloid inhibitory peptides, and to pharmaceutical compositions comprising such peptides, for use in methods of treating or preventing or delaying the onset of synucleinopathies, in particular of Parkinson's disease (PD) or dementia with Lewy bodies. Furthermore, the present invention relates to such peptides, in particular such amyloid inhibitory peptides, for use in methods of diagnosing such synucleinopathies. Furthermore, the present invention also relates to a kit for the in-vitro or in-vivo detection and, optionally, quantification of amyloidogenic polypeptides, amyloid fibrils or amyloid aggregates, and/or for the diagnosis of synucleinopathies in a patient.

Amyloid self-assembly is linked to devastating cell-degenerative diseases including Alzheimer's disease (AD), type 2 diabetes (T2D), Parkinson's disease (PD) and dementia with Lewy bodies, amongst others. Molecules blocking amyloidogenesis of the key amyloid polypeptides of AD and T2D, i.e. amyloid-β peptide (Aβ40(42)) (AD) and islet amyloid polypeptide (IAPP) (T2D), or of synucleinopathies, such as Parkinson's disease (PD) or dementia with Lewy bodies, i.e. α-synuclein (αSyn), could thus become drug candidates. However, the rational design of amyloid inhibitors is a difficult task. Major reasons are the high conformational flexibility of most amyloidogenic polypeptides and several amyloidogenic proteins, high affinity interactions of amyloid self-assembly, and the large size of involved interfaces. Additional challenges include a low blood-brain-barrier (BBB) permeability, high production costs, and potential immunogenicity of antibodies, low proteolytic stability and usually no BBB crossing of linear peptides, while small molecules often lack high affinity and specificity and cannot block interactions involving large interfaces. Importantly, none of the reported inhibitors of amyloid self-assembly of α-synuclein (αSyn), Aβ40(42) or IAPP has yet advanced to the clinic.

Synucleinopathies, also sometimes termed "a-synucleinopathies" are neurodegenerative diseases characterized by the abnormal accumulation of aggregates of a pre-synaptic protein of 140 residues, termed "a-synuclein" (αSyn) and of several mutants thereof. Parkinson's disease (PD) is the most prominent synucleinopathy and is the second most common neurodegenerative disease (after Alzheimer's disease) and affects more than 10 million people worldwide.

Patients with synucleinopathies have features of parkinsonism, impaired cognition, sleep disorders and visual hallucinations. Parkinsonism is characterized by tremor, bradykinesia, rigidity and postural instability.

Previously, it was surmised that type 2 diabetes (T2D) is a risk factor for Parkinson's disease. IAPP fibrils had previously been shown to be able to act as cross-seeding nuclei for amyloid self-assembly of α-synuclein (αSyn). However, both α-synuclein (αSyn) and IAPP are intrinsically disordered proteins, and possible cross-interaction sites and structures of possible hetero-assemblies are unknown, making the design of amyloid inhibitors extremely difficult.

US 7,745,490 discloses substituted N-aryl benzamides as potential inhibitors of fibril formation of α-synuclein (αSyn), Aβ peptide(s), IAPP and others. It is not clear however, whether these inhibitors actually do inhibit such fibril formation, and whether they might also be able to interfere with cross-seeding caused by interactions of pre-existing minute amounts of fibrillar aggregates. Of note, there are no reports so far about molecules which are able to block IAPP/aSyn cross-seeding interactions.

Furthermore, none of the reported α-synuclein (αSyn) amyloid inhibitors or pipeline therapeutics for Parkinson's disease, including antibodies, peptides and small molecules, have yet advanced into the clinic or have been shown to suppress cross-seeding of α-synuclein (αSyn). Parkinson's disease and any other synucleinopathy are still incurable diseases.

Accordingly, there is a need for new inhibitors of amyloid self-assembly of α-synuclein (αSyn) and/or inhibitors that would interfere with a possible interaction between α-synuclein (αSyn) and IAPP. There is furthermore a need to provide amyloid inhibitors that inhibit amyloid self-assembly of α-synuclein (αSyn). There is also a need to provide amyloid inhibitors that inhibit a cross-seeding of α-synuclein (αSyn) assembly by IAPP.

In a first aspect, the present invention relates to a peptide, preferably an amyloid inhibitory peptide, having an amino acid sequence according to formula 0

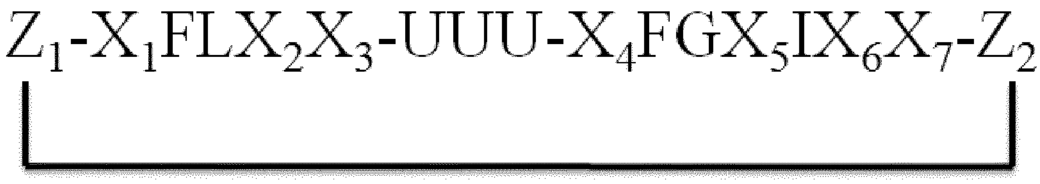

(Formula 0)

wherein

Z1 and Z2 are selected from the following pairs

a) cysteine and cysteine,
b) aspartic acid and lysine, or lysine and aspartic acid,
c) aspartic acid and ornithine, or ornithine and aspartic acid,
d) aspartic acid and 2,4-diaminobutyric acid, or 2,4-diaminobutyric acid and aspartic acid,
e) aspartic acid and 2,3-diaminopropionic acid, or 2,3-diaminopropionic acid and aspartic acid,
f) glutamic acid and lysine, or lysine and glutamic acid,
g) glutamic acid and ornithine, or ornithine and glutamic acid,
h) glutamic acid and 2,4-diaminobutyric acid, or 2,4-diaminobutyric acid and glutamic acid,
i) glutamic acid and 2,3-diaminopropionic acid, or 2,3-diaminopropionic acid and glutamic acid;

with denoting a covalent bond between Z1 and Z2, thus providing for a cyclization of the peptide;

X1, X2, X3, X4, X5, X6, and X7 are, independently at each occurrence, selected from glycine, asparagine, valine, histidine, leucine, serine, alanine, and threonine;

F is, independently at each occurrence, phenylalanine;

L is leucine;

U is, independently at each occurrence, selected from arginine, homoarginine, citrulline, ornithine, lysine, and norleucine;

G is glycine;

I is isoleucine;

wherein Z1, Z2, X1-X7, F, L, U, G and I are L-amino acid residues or D-amino acid residues, or some of Z1, Z2, X1-X7, F, L, U, G and I are L-amino acid residues and others are D-amino acid residues;

and pharmaceutically acceptable salts, esters, solvates, polymorphs and modified forms thereof;

wherein preferably said peptide has an amino acid sequence according to formula 0a

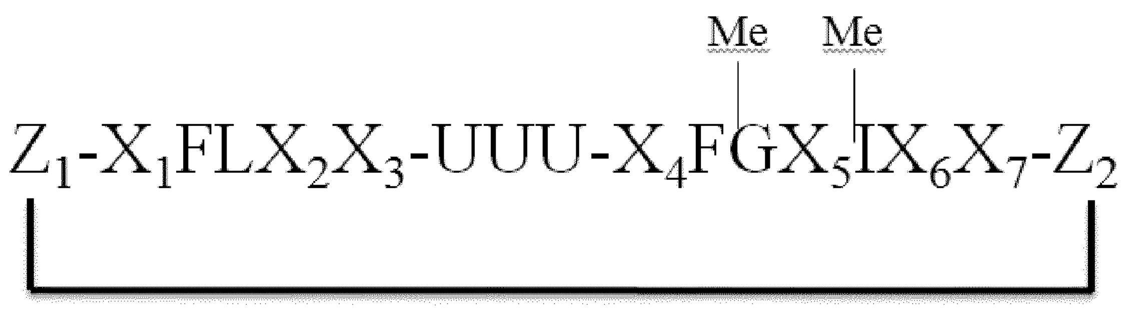

(Formula 0a)

wherein

Z1, Z2, X1-X7, F, L, U, G, I are as defined above, and

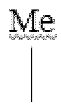

is N-methyl,

for use in a method of treating, or preventing, or delaying the onset and/or pathogenesis of, a synucleinopathy.

In one embodiment, the peptide according to the present invention, preferably the amyloid inhibitory peptide according to the present invention, has an amino acid sequence according to formula 1

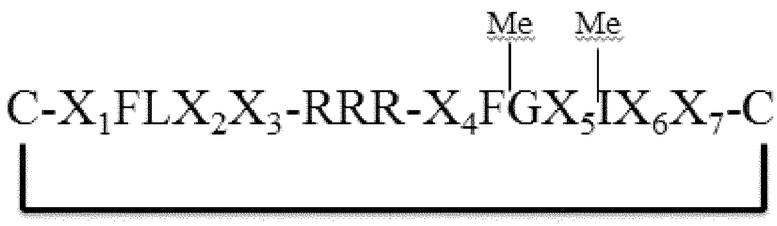

(Formula 1)

or an amino acid sequence according to formula 1*

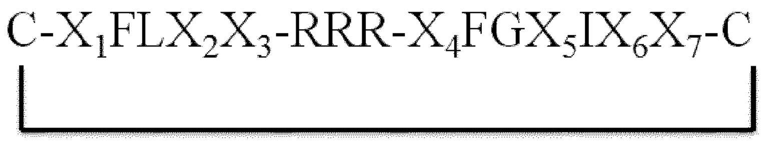

(Formula 1*)

wherein

C is cysteine;
X1, X2, X3, X4, X5, X6, and X7 are, independently at each occurrence, selected from glycine, asparagine, valine, histidine, leucine, serine, alanine, and threonine;
F is, independently at each occurrence, phenylalanine;
L is leucine;
R is arginine;
G is glycine;
I is isoleucine;

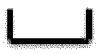 is a disulfide bond;

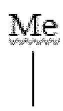

is N-methyl;
C, X1-X7, F, L, R, G and I are L-amino acid residues or D-amino acid residues, or some of C, X1-X7, F, L, R, G and I are L-amino acid residues and others are D-amino acid residues;

and pharmaceutically acceptable salts, esters, solvates, polymorphs and other modified forms thereof.

In an embodiment of the peptide, either

a) X1 and X4 are asparagine, X2 is valine, X3 is histidine, X4 is glycine, X5 is glycine, X6 and X7 are glycine;
b) X1-X7 are glycine, alanine or serine;
c) X1-X7 are glycine;
d) X1-X3 are glycine, X4 is asparagine, X5 is alanine, and X6-X7 are glycine; or
e) X1-X3 are glycine, X4 is asparagine, X5 is alanine, X6 is leucine, X7 is serine.

In one embodiment, Z1, Z2, C, X1-X7, F, L, U, R, G, and I are L-amino acid residues.
In one embodiment, R is, at each occurrence, D-arginine, and/or

F is, at each occurrence, D-phenylalanine, and/or

L is D-leucine, and/or

Z1, Z2 and C are D-amino acid residues, and/or

I is D-isoleucine or N-methyl-D-isoleucine.

In one embodiment, the peptide according to the present invention has a sequence according to a formula selected from the following formulae 2a - 2e, 2a* - 2e*:

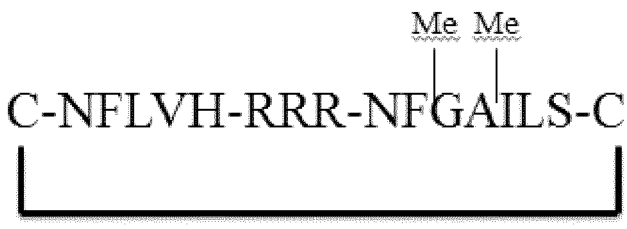

(Formula 2a)

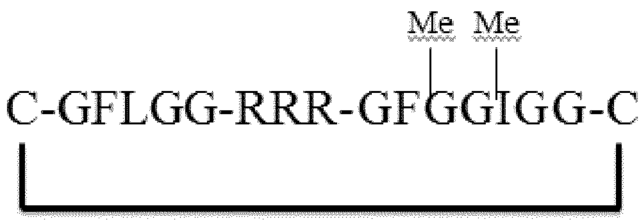

(Formula 2b)

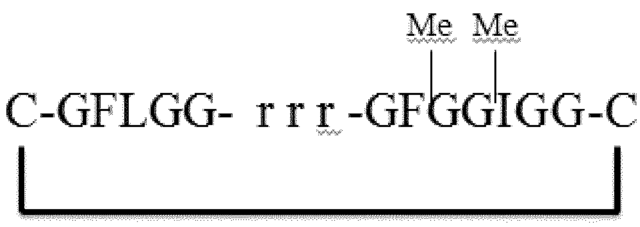

(Formula 2c)

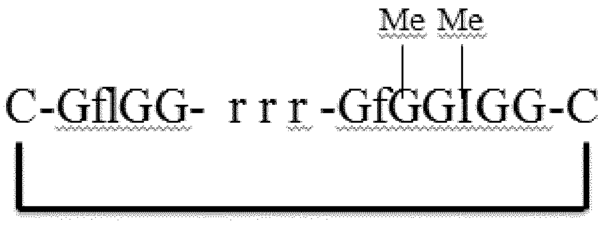

(Formula 2d)

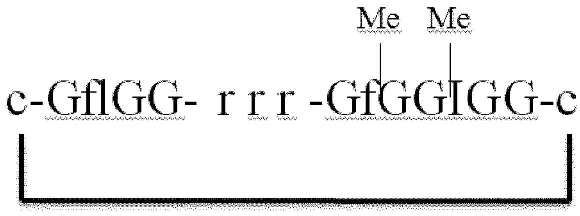

(Formula 2e)

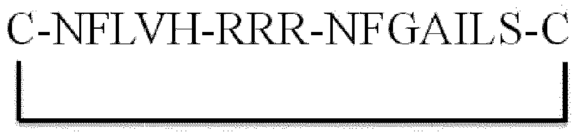

(Formula 2a*)

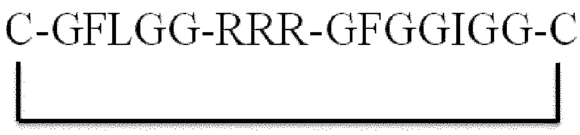

(Formula 2b*)

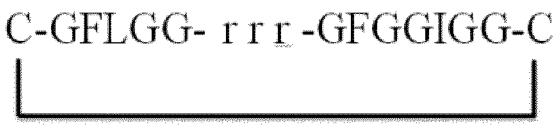

(Formula 2c*)

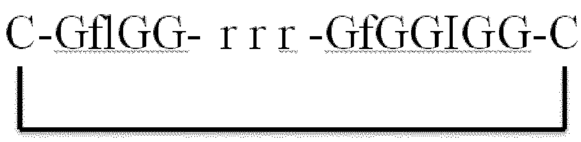

(Formula 2d*)

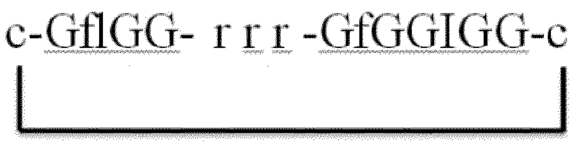

(Formula 2e*)

wherein upper case letters represent L-amino acid residues or D-amino acid residues, preferably L-amino acid residues, and lower case letters represent D-amino acid residues.

In a particularly preferred embodiment, the peptide(s) according to the present invention have a sequence according to a formula selected from 2b, 2e, 2b* and 2e*

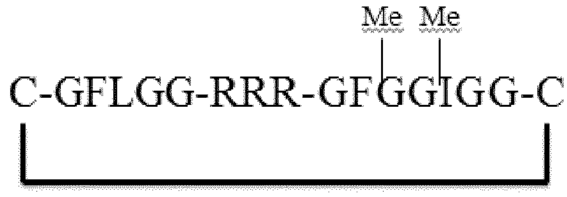

(Formula 2b)

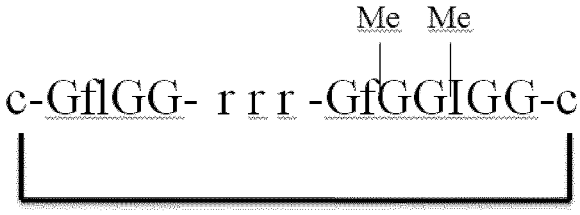

(Formula 2e)

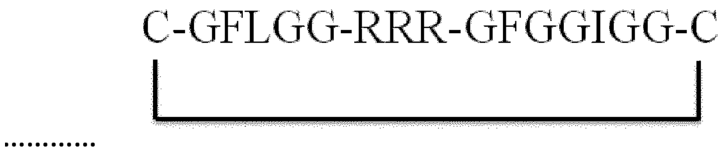

(Formula 2b*)

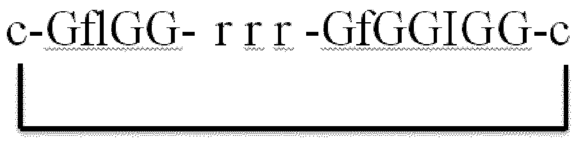

(Formula 2e*),

wherein upper case letters represent L-amino acid residues or D-amino acid residues, preferably L-amino acid residues, and lower case letters represent D-amino acid residues.

In one embodiment, said peptide consists of a sequence according to any of formulae o, oa, 1, 1*, 2a - 2e, 2a* - 2e*, as defined above, respectively, preferably of a sequence according to any of formulae 2b, 2e, 2b* and 2e*, as defined herein.

In one embodiment, said synucleinopathy is selected from Parkinson's disease (PD), dementia with Lewy bodies, multiple system atrophy, mitochondrial membrane protein associated neurodegeneration, and synucleinopathy-related comorbidities, including Parkinson's disease (PD)/Alzheimer's disease (AD), and Parkinson's disease (PD)/type 2 diabetes (T2D), wherein preferably, said synucleinopathy is Parkinson's disease (PD).

In one embodiment, said peptide is an amyloid inhibitory peptide that preferably binds to α-synuclein (αSyn), preferably to monomers and/or oligomers and/or fibrils thereof.

In a particularly preferred embodiment, which may be combined with any other embodiment herein, said peptide binds to α-synuclein (αSyn), preferably to monomers and/or oligomers and/or fibrils thereof, with nanomolar affinity. In a particularly preferred embodiment, said peptide binds to monomers of α-synuclein (αSyn) with nanomolar affinity.

In one embodiment, which may also be combined with any other embodiment herein, said peptide binds to IAPP, preferably to monomers and/or oligomers and/or fibrils thereof. In a particularly preferred embodiment, said peptide binds to IAPP with nanomolar affinity.

In one embodiment, which may also be combined with any other embodiment herein, said peptide binds to

Abeta40(42), preferably to monomers and/or oligomers and/or fibrils thereof. In a particularly preferred embodiment, said peptide binds to Abeta40(42) with nanomolar affinity.

It should be noted that in preferred embodiments, where reference is made to a "peptide" in general, such peptide may also be referred to as an "amyloid inhibitory peptide". An "amyloid inhibitory peptide" is a peptide that functions as, or can be used as, an "amyloid inhibitor".

Without wishing to be bound by any theory, the present inventors believe that the amyloid inhibitory effect of the amyloid inhibitory peptides described herein is mainly mediated by their binding to key amyloid polypeptides, in particular to α-synuclein (αSyn) and/or to islet amyloid polypeptide (IAPP), more specifically to specific sites of α-synuclein (αSyn). The present inventors furthermore believe, again without wishing to be bound by any theory, that these specific sites mediate both self-assembly of α-synuclein (αSyn) and its possible interactions with islet amyloid polypeptide (IAPP), thus interfering with self-seeding interactions of α-synuclein (αSyn) with itself, but also its cross-interactions and cross-seeding by IAPP. Their potent (because of nanomolar IC50 values (Table 1)) amyloid inhibitory effects are also related to their ability to bind with nanomolar affinity to both α-synuclein (αSyn) and IAPP, in their various conformations (monomeric, oligomeric, or fibrils) and to interfere with their interactions and/or self-assembly.

Hence, the term "amyloid inhibitory" as used herein in the context of a peptide, refers to the capability of such peptide to block or inhibit amyloid self-assembly or amyloidogenesis or aggregation or amyloid formation, preferably of the key amyloid polypeptide(s) believed to be responsible for synucleinopathies, in particular of α-synuclein (αSyn), but also of islet amyloid polypeptide (IAPP), if/when such IAPP interacts with α-synuclein (αSyn). By binding with high affinity specific αSyn segments or "sites" which are crucial for aSyn self-assembly and its interactions with IAPP, the amyloid inhibitory peptides of the invention are thus capable of suppressing or blocking both self-seeded and cross-seeded amyloid self-assembly of αSyn while their ability to interact with or block amyloid self-assembly of IAPP likely contributes to their potent amyloid inhibitor function.

In one embodiment, the peptide(s) according to the present invention binds(bind) to at least one of the following stretches of the amino acid sequence of α-synuclein (αSyn): αSyn(1-14), αSyn(34-52), and αSyn(87-105), preferably to at least two of these stretches, more preferably all three of them.

The terminology "αSyn(x-y)", as used in this context is meant to refer to a stretch of the amino acid sequence of α-synuclein (αSyn), as shown in Example 1.16 herein, with "x" denoting the first (N-terminal) residue and "y" denoting the last (C-terminal) residue of such stretch.

Amyloid inhibitory peptides in accordance with the present invention are useful as amyloid inhibitors, and may thus be used for therapeutic and/or diagnostic purposes, i.e. they may be used for treatment and/or diagnosis of diseases involving amyloid self-assembly or amyloidogenesis, in particular of synucleinopathies. Preferably such synucleionpathies are selected from Parkinson's disease (PD), dementia with Lewy bodies, multiple system atrophy, mitochondrial membrane protein associated neurodegeneration, and synucleinopathy-related comorbidities, including Parkinson's disease (PD)/Alzheimer's disease (AD), and Parkinson's disease (PD)/type 2 diabetes (T2D), wherein preferably, said synucleinopathy is Parkinson's disease (PD).

In a further aspect, the present invention also relates to a composition comprising a peptide, preferably an amyloid inhibitory peptide, according to the present invention, and a suitable solvent, such as water, and a buffer.

In a further aspect, the present invention also relates to a pharmaceutical composition comprising a peptide, preferably an amyloid inhibitory peptide, according to the present invention and a pharmaceutically acceptable excipient, for use in a method of treating or preventing or delaying the onset of a synucleinopathy selected from Parkinson's disease (PD), dementia with Lewy bodies, multiple system atrophy, mitochondrial membrane protein associated neurodegeneration, and synucleinopathy-related comorbidities, including Parkinson's disease (PD)/Alzheimer's disease (AD), and Parkinson's disease (PD)/type 2 diabetes (T2D), wherein preferably, said synucleinopathy is Parkinson's disease (PD).

In such pharmaceutical composition, the peptide may occur as such, or it may be linked to other entities/molecules that endow the peptide with a specific functionality. For example, there may be a tag attached to increase blood-brain-barrier permeability, or it may be attached to a specific reporter molecule, such as a dye or a quantum dot, allowing the detection in diagnostic methods (preferably whilst retaining the therapeutic functionality of the peptide - "theranostic applications"). The use of quantum dots may be particularly useful in various imaging technologies, such as MRI, PET, PET-MRI, or specifically quantum-dot-based brain imaging methodologies. In certain embodiments, the peptide may be attached to a nanoparticle, to a suitable carrier molecule, to a targeting entity or other functional molecule.

Because the peptides according to the present invention, however, are indeed capable of passing the blood-brain-barrier, the present invention also relates to the use of a peptide according to the present invention, as defined above, as a carrier for molecules, substances or compounds to pass the blood-brain-barrier. According to this aspect, in certain embodiments, the molecule to pass the blood-brain-barrier is linked, preferably covalently linked, to a peptide according to the present invention as defined above.

In one embodiment of such peptide or composition for use, said method comprises administering an effective amount of said peptide or of said composition to a patient in need thereof.

In a further aspect, the present invention also relates to a peptide, in particular an amyloid inhibitory peptide,

according to the present invention, or the pharmaceutical composition according to the present invention as defined above, for use in a method of diagnosing a synucleinopathy which is selected from Parkinson's disease (PD), dementia with Lewy bodies, multiple system atrophy, mitochondrial membrane protein associated neurodegeneration, and synucleinopathy-related comorbidities, including Parkinson's disease (PD)/Alzheimer's disease (AD), and Parkinson's disease (PD)/type 2 diabetes (T2D), wherein preferably, said synucleinopathy is Parkinson's disease (PD).

In one embodiment of such peptide or composition for use, said method comprises administering an effective amount of said peptide or of said composition to a subject to be tested for a synucleinopathy.

In one embodiment of such peptide or composition for use, said peptide, in particular said amyloid inhibitory peptide, is linked to or administered together with a suitable reporter molecule that allows detection of α-synuclein (αSyn) and/or amyloid and/or non-amyloid aggregates and/or co-aggregates (e.g. aSyn/IAPP or aSyn/Abeta) and/or fibrils thereof, by a suitable detection methodology, such as positron emission tomography (PET), nuclear magnetic resonance (NMR), magnetic resonance imaging (MRI), and PET-MRI and said subject, after administration of said peptide, is subjected to PET, NMR, MRI, PET-MRI.

In a yet a further aspect, the present invention relates to a kit for the in-vitro or in-vivo detection and/or quantification of α-synuclein (αSyn) and/or amyloid and/or non-amyloid aggregates and/or co-aggregates (e.g. aSyn/IAPP or aSyn/Abeta) and/or fibrils thereof, or for the diagnosis of a synucleinopathy selected from Parkinson's disease (PD), dementia with Lewy bodies, multiple system atrophy and mitochondrial membrane protein associated neurodegeneration, wherein preferably, said synucleinopathy is Parkinson's disease (PD), in a patient. Preferably, said kit comprises a peptide, in particular an amyloid inhibitory peptide according to the present invention as defined herein, in a freeze-dried form in a suitable container, a buffered solvent in a separate container for reconstitution of said peptide in solution, and, optionally, means to dispense said peptide once reconstituted in solution, such as a syringe or pipette. Alternatively, the kit may contain the peptide, in particular the amyloid inhibitory peptide according to the present invention as defined above, in an already reconstituted, ready-to-use form.

In a yet a further aspect, the present invention also relates to the use of the peptide according to the present invention, in an in-vitro assay, such as an enzyme linked immunosorbent assay (ELISA) or a radioimmuno assay (RIA), for the detection of α-synuclein (αSyn) monomeric or oligomeric aggregates and/or amyloid and/or non-amyloid aggregates and/or co-aggregates (e.g. aSyn/IAPP or aSyn/Abeta) and/or fibrils thereof.

Such use may, in certain embodiments, involve the analysis of body fluids, including but not limited to blood, cerebrospinal fluid, lymph, urine, and other body fluids or it may involve the analysis of brain biopsies, and may also further involve the use of suitable reporter molecules to which the peptide may be attached or with which the peptide may be used together.

In a further aspect, the present invention relates to the use of a peptide according to the present invention, as defined above, for the manufacture of a medicament for the treatment, prevention, delay of onset of, or diagnosis of a synucleinopathy selected from Parkinson's disease (PD), dementia with Lewy bodies, multiple system atrophy and mitochondrial membrane protein associated neurodegeneration, wherein preferably, said synucleinopathy is Parkinson's disease (PD).

In yet a further aspect, the present invention also relates to a method of treatment, prevention, delay of onset of, or diagnosis of a synucleinopathy selected from Parkinson's disease (PD), dementia with Lewy bodies, multiple system atrophy and mitochondrial membrane protein associated neurodegeneration, wherein preferably, said synucleinopathy is Parkinson's disease (PD), wherein said method comprises administering an effective amount of said peptide or of said composition according to the present invention as defined herein, to a patient in need thereof or to a subject to be tested.

The present inventors have provided cyclic peptides which function as nanomolar inhibitors of amyloid self-assembly of α-synuclein (αSyn) and which therefore have manifold applications.

Moreover, these peptides bind, with high (i.e. nanomolar) affinity, to α-synuclein (αSyn) monomers and/or amyloid aggregates, in particular to monomers thereof. Moreover, the peptides "detoxify" oligomers of α-synuclein (αSyn) (as for example shown in the ex-vivo assay of figure 4 as well as in figure 6.)

In the present application, use is made of the one-letter-code for amino acid residues and the three-letter-code for amino acid residues. Hence, amino acid residues are designated herein by reference to their respective one-letter-code or three-letter-code. Accordingly, alanine is A or Ala; arginine is R or Arg; asparagine is N or Asn; aspartic acid is D or Asp; cysteine is C or Cys; glutamine is Q or Gln; glutamate is E or Glu; glycine is G or Gly; histidine is H or His; isoleucine is I or Ile; leucine is L or Leu; lysine is K or Lys; methionine is M or Met; phenylalanine is F or Phe; proline is P or Pro; serine is S or Ser; threonine is T or Thr; tryptophan is W or Trp; tyrosine is Y or Tyr; valine is V or Val.

Sometimes, in this application, reference to amino acid sequences is made by reciting the individual residues. Where such amino acid sequence is indicated by using upper case letters only, this means that these amino acids are unspecified in terms of their chirality, i.e. the residues may be L-amino acids or D- amino acids or a mixture of the two possibilities, i.e. some of the residues in the sequence may be L-amino acids and others may be D- amino acids. In one embodiment, the upper case amino acids may be all L-amino acids.

In those instances, where such amino acid sequence is indicated by using upper case letters and lower case

letters together in one sequence, this means that the upper case amino acids may be L-amino acids or D- amino acids, preferably L-amino acids, and the lower case amino acids are, in any case, D- amino acids.

Moreover, sometimes, in this application, reference to amino acid sequences is made by reciting the individual residues as free amino acids, such as "glycine", "glutamic acid", etc., notwithstanding the fact that these residues appear in the respective amino acid sequence in their respective covalently linked form, i.e. with the individual residues linked by appropriate peptide bonds, i.e. amide bonds, between them.

The term "N-methyl" or "NMe" or "

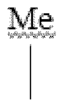

", as used herein, refers to a methyl group that is attached to the nitrogen in the amide bond between two amino acid residues.

For example where a sequence is indicated as

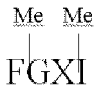

this means that a methyl group is attached to the amide nitrogen forming the amide bond between F and G, and a further methyl group is attached to the amide nitrogen forming the amide bond between X and I. This may also be referred to as "N-methylated glycine" and "N-methylated isoleucine" respectively, or "methylated glycine" and a "methylated isoleucine", respectively, because the respective amide nitrogen belongs to glycine and isoleucine respectively in these cases.

In some preferred embodiments of the peptide according to the present invention, there is a methyl group attached to the amide bond between F11 and G12 (i.e. a "methylated glycine 12"), and a further methyl group attached to the amide bond between X13 and I14 (i.e. a "methylated isoleucine 14"), if one uses a numbering which is based on a 17-peptide according to any of formulae 0, 0a, 1, 1*, 2, 2a-2e, 2a*-2e*. By reference to the IAPP-numbering, these positions correspond to the amide bond between F23 and G24 and the amide bond between A25 and I26.

The symbol 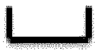 refers to a covalent bond between two residues thus linked. For example, if the two residues thus linked are two cysteines, it means a disulfide bond.

Alternatively, it may mean a lactam bridge involving the sidechains of the respective two amino acids. For example, the sidechains of aspartic acid and lysine, or of glutamic acid and lysine may form such a lactam bridge. Pairs that may be involved in such lactam bridge formation are Asp-Lys, Asp-Orn, Asp-Dab (Dab meaning 2,4 diaminobutyric acid), Asp-Dap (Dap meaning 2,3-diaminopropionic acid), Glu-Lys, Glu-Orn, Glu-Dab, Glu-Dap, or pairs with an inverse arrangement of the aforementioned residues. In one embodiment, it also possible to obtain a cyclisation via the generation of 1,2,3-triazole rings, generated by click reactions between specific amino acids, instead of cysteines.

In one embodiment, the N-terminus and/or the C-terminus of the peptides according to the present invention are protected. Suitable protecting groups are manifold and are known to a person skilled in the art. For example, the N-terminus may be acetylated or formylated, or there may be an even longer chain attached such as palmitoyl. The C-terminus could be protected via formation of an amide or carbonic acid ester.

In one embodiment, the C-termini of the peptide(s) according to the present invention, in particular of the amyloid inhibitory peptides according to the present invention, more particularly of the peptides according to formulae 0, 0a, 1, 1*, 2a-2e, 2a*-2e* according to the present invention, are protected by an amide. In other embodiments, the C-terminus is in its free carboxy-form; in yet other embodiments, it is in esterified form. In particularly preferred embodiments, the C-termini of the peptide(s) according to the present invention, in particular of the amyloid inhibitory peptides according to the present invention, more particularly of the peptides according to formulae 0, 0a, 1, 1*, 2a-2e, 2a*-2e* according to the present invention, are protected by an amide, and the respective N-termini of the peptides are unprotected, i.e. in their $NH_2$-form (or $NH_3^+$-form).

The term "treatment" or "treating", as used herein, encompasses both prophylactic treatment and therapeutic treatment. In a preferred embodiment, it specifically refers to therapeutic treatment.

The present inventors have managed to design peptidic inhibitors of amyloid self-assembly of α-synuclein (αSyn) and of amyloid fibril formation of α-synuclein (αSyn) which is cross-seeded by IAPP fibrils. Without wishing to be bound by any theory, the present inventors believe that the peptidic inhibitors according to the present invention (which mimic IAPP surfaces that interact with IAPP, Aβ or α-synuclein (αSyn)) target interaction surfaces of α-synuclein (αSyn) that are responsible for self-aggregation and/or for interaction and/or for co-aggregation with other surfaces or entities,

such as IAPP, while maintaining only minimal IAPP-derived self/cross-recognition elements. In preferred embodiments, the peptides reported herein also exhibit both strongly improved proteolytic stability in human plasma and blood-brain-barrier crossing ability in a cell model.

Furthermore, reference is made to the figures, wherein

Figure 1 shows the inventors' strategy for identification of IAPP regions that interact with α-synuclein (αSyn) by using peptide arrays (a) and determination of the binding affinities of interactions of α-synuclein (αSyn) with IAPP and IAPP(8-28) by fluorescence spectroscopic titrations (b,c). a) Synthetic peptide arrays containing IAPP decamers (bold & underlined) were incubated with Biotin-α-synuclein (αSyn) (0.5 μM); decamers which bound Biotin α-synuclein (αSyn) are in dashed rectangles. Array representative of two arrays synthesized in parallel and two independent incubations with Biotin-α-synuclein (αSyn). b,c) Fluorescence emission spectra of Fluos-IAPP (=fluorescein-labelled IAPP) (b) and Fluos-IAPP(8-28) (c) (5 nM) alone or their mixtures with various molar ratios of α-synuclein (αSyn) (Fluos-peptide/ α-synuclein (αSyn)) as indicated; data from 1 representative assay out of 3. Insets show binding curves; data means (±SD) of 3 titration assays.

Figure 2 shows the effects of **2b, 2e,** and **4Ala-2b** on non-seeded (a-c), seeded with preformed fibrillary αSyn (fαSyn) (d-f), and fibrillary IAPP (fIAPP)-cross-seeded αSyn amyloid self-assembly and related cell-damaging effects (g-i). a-c) Fibrillogenesis of αSyn (3 μM) alone or in the presence of **2b** and **2e** (1/1) or **4Ala-2b** (1/50) determined by ThT binding (means (±SD), 3 assays (3 wells each)) (a); TEM images of solutions (7 day-aged) from (a) as indicated (color code as in (a)) (scale bars, 100 nm) (b); PC12 cell viability after treatment with solutions from (a) (7 day-aged) determined by the MTT reduction assay (means (±SD), 3 assays (3 wells each)) (c). d-f) Fibril formation of αSyn alone (3 μM) or seeded by preformed fαSyn (10%) alone or with **2b** and **2e** (1/1) or **4Ala-2b** (1/50) as determined by the ThT binding assay and ThT binding of fαSyn seeds (0.3 μM) (means (±SD), 3 assays (3 wells each) (d); TEM images of solutions from (d) aged for 7 days (seeded αSyn /**2b(2e)** mixtures) or for 24 h (seeded αSyn alone or with **4Ala-2b**) and from fαSyn seeds (scale bars, 100 nm) (e); PC12 cell viability after treatment solutions from (d) (7 day-aged) determined by the MTT reduction assay (means (±SD), 3 assays (3 wells each)) (f). g-i) Fibrillogenesis of αSyn (3 μM) alone or cross-seeded by fIAPP (10%) alone or with **2b** and **2e** (1/1) or **4Ala-2b** (1/50) determined by ThT binding and ThT binding of fIAPP seeds (0.3 μM) (means (±SD), 3 assays (3 wells each)) (g); TEM images of solutions from (g) aged for 7 days (cross-seeded αSyn**/2b(2e))** or for 24 hours (cross-seeded αSyn alone or with **4Ala-2b)** as indicated (scale bars, 100 nm) (h); PC12 cell viability after treatment with solutions from (g) (7 day-aged) determined by the MTT reduction assay (means (±SD), 3 assays (3 wells each).

Figure 3 shows studies on interactions, hetero-complexes, and mechanism of inhibitory effects of **2e** and **2b** on αSyn amyloid self-assembly. a,b) Left, app. $K_d$s of interactions of Fluos-**2b** (a) and Fluos-**2e** (b) with αSyn determined by fluorescence spectroscopic titrations. Fluorescence emission spectra of Fluos-**2b** or Fluos-**2e** (1 nM) and their mixtures with various molar ratios of αSyn are shown as indicated; spectra from 1 representative binding assay out of 3. Right side, binding curves; data means (±SD) of 3 titration assays; app. Kas in Table 2. c,d) Far-UV CD spectra of αSyn (1 μM) alone and its mixtures with **2b** (c) or **2e** (d) (10 μM) measured at 0 h and after 48 h of incubation, e) Characterization of αSyn/MCIP hetero- and αSyn homo-oligomers by cross-linking with glutaraldehyde, SDS-PAGE, and Western blot with anti- αSyn (left) or anti-**2e(2b)** (right) antibodies (αSyn, 10 μM; MCIPs, 50 μM); representative results from 3 assays. f) Characterization of αSyn/**2e** hetero-complexes in comparison to αSyn and **2e** alone by size exclusion chromatography (SEC). Chromatograms of αSyn (3 μM), **2e** (30 μM), and the αSyn/**2e** mixture (1/10) are shown. Inset, ESI-MS spectrum (deconvoluted) of the 21 min peak from SEC of the αSyn/**2e** mixture. Determined MWs as indicated; calculated mass (average) 14460.27 Da (αSyn) and 1695.04 Da **(2e).** Representative results from 3 SEC analyses and ESI-MS. g) Kinetics of αSyn self-assembly into An-reactive toxic oligomers alone or in the presence of **2e** followed by slot blot analysis using the A11 antibody. Solutions αSyn (3 μM) alone, αSyn/**2e** (1/1), and **2e** (3 μM) alone were analyzed at indicated incubation time points. Representative results from 4 assays. h) Binding of **2b, 2e,** and **4Ala-2b** to fαSyn and fIAPP determined by dot blot analysis. Fluos-**2b**, Fluos-**2e**, and Fluos-**4Ala-2b** (1.5 μM) and the buffer alone control were incubated with membranes containing spotted fαSyn or fIAPP; binding visualized by fluorescence. Representative results from 3 assays.

Figure 4 shows the suppression of αSyn oligomer-induced LTP impairment in murine hippocampal slices *ex-vivo* by **2b** and **2e.** a,b) Time course of synaptic transmission (fEPSP, field excitatory postsynaptic potential) after treatment with a) aCSF medium (buffer control), αSyn oligomers (175 nM), **2b** alone (1.75 μM), and αSyn oligomers**/2b** mixture (1/10) or b) aCSF medium, αSyn oligomers (175 nM), **2e** (1.75 μM), and αSyn oligomers/**2e** mixture (1/10); data means (±SD), from n=10 samples/treatments each. c) LTP values: bars show the averages from the last 10 min of recording; data means (±SD), n=10 for each group; p-values as indicated; calculated using non-parametric testing with Mann-Whitney U-tests or a Kruskal-Wallis test.

Figure 5 shows the identification of αSyn segments mediating its interactions with MCIPs and IAPP by synthetic peptide arrays (a,e), determination of binding affinities by fluorescence spectroscopic titrations (b-d), and overview of key αSyn interaction sites and related functions (f). a) Identification of key αSyn regions interacting with **2e** using peptide arrays. Top, αSyn sequence; identified **2e-**binding regions highlighted in orange; colored arrows indicate β-strands β1- β8 in the fαSyn fold. Bottom, peptide array containing αSyn decamers following incubation with Fluos-**2e** (1μM) and bound peptide visualization by fluorescence; identified Fluos-**2e**-binding segments αSyn(1-14), αSyn(34-52), and αSyn(87-105) in orange rectangles. Array representative of 2 arrays synthesized in parallel and 2 independent incubations with Fluos-**2e** (data not shown). b-d) Determination of app. Kas of Fluos-**2e** interactions with identified **2e**-binding αSyn segments by fluorescence spectroscopic titrations. Left, fluorescence emission spectra of Fluos-**2e** alone (5 nM) or with various molar ratios of αSyn(1-14) (b), αSyn(34-52) (c), and αSyn(87-105) (d) (Fluos-**2e**/peptide as indicated). Spectra from 1 representative assay out of 3. Right, binding curves; data means (±SD) of 3 assays (see Table 2). e) Identification of key αSyn regions interacting with IAPP using peptide arrays. Top, αSyn sequence; identified IAPP-binding regions highlighted in pink; colored arrows as under (a).Bottom, peptide array containing αSyn decamers (as in (a)) following incubation with Fluos-IAPP (1 μM) and visualization. The identified 3 major Fluos-IAPP-binding segments αSyn(1-13), αSyn(34-46), and αSy(87-104) are in pink rectangles made by solid lines; the weaker binding αSyn(68-80) is in a pink rectangle made by dashed lines. Array representative of two arrays synthesized in parallel and two independent incubations with Fluos-IAPP (data not shown). f) Schematic overview of the 3 identified key αSyn segments mediating its interactions with **2e, 2b**, and IAPP and previously reported interactions & functions of related αSyn sequence parts. White arrows indicate β-strands β1- β8 in the fαSyn fold.

Figure 6 demonstrates a confirmation of the presence of cell-damaging αSyn oligomers in solutions used for studying effects of MCIPs on hippocampal synaptic LTP impairment mediated by αSyn oligomers (see Fig. 4). αSyn oligomers were prepared according to a previously described protocol (M. J. Diogenes, et al., *Journal of Neuroscience* **2012,** *32*, 11750-11762.). Following a 5-day aging of αSyn (138 μM) at 37°C, αSyn oligomers were separated from fibrils by centrifugation and supernatants were used for the LTP impairment studies. The presence of cytotoxic oligomers in the supernatant fraction was confirmed by the ThT binding assay (a), TEM (b), dot blot with the anti-oligomer antibody A11 (c), and the MTT reduction assay (d); for comparison, freshly dissolved αSyn (αSyn monomers) or buffer alone and pellet fractions (fibrils) were studied as well. a) ThT binding properties of αSyn monomers and of the supernatant (αSyn oligomers) and pellet (αSyn fibrils) fractions (αSyn 1.7 μM) as indicated. Data is means (±SD) from 3 αSyn oligomer preparations. b) TEM micrographs of supernatant (αSyn oligomers) and pellet (αSyn fibrils) fractions; scale bars 1 μm and 100 nm, respectively, c) Dot blot analysis of A11 antibody binding of supernatant (αSyn oligomers) and pellet (αSyn fibrils) fractions as compared to αSyn monomers (5 μg each); a representative dot blot (n=2) is shown. d) Effects of buffer, αSyn monomers, and the supernatant fraction (αSyn oligomers) (αSyn, 300 nM) on PC12 cell viability determined by MTT reduction assay. Data means (±SD) of 4 assays (n=3 wells each); statistical analysis by one-way ANOVA with post-hoc Tukey test.

Moreover, reference is made to the following examples which are given to illustrate and not to limit the present invention.

## Examples

### Example 1 - Materials and Methods

#### 1. Peptides, peptide synthesis, and proteins

All peptides, i.e. macrocyclic inhibitory peptides (MCIPs), **4Ala-2b,** ISMs, IAPP, and IAPP or αSyn segments, and their $N^{\alpha}$-terminal fluorescein-labeled analogs (Fluos-peptide) (all C-terminal amides) were synthesized using Fmoc-based solid phase synthesis (SPPS) on Rink resin and purified via RP-HPLC. Briefly, double or triple couplings were performed using Fmoc-protected amino acids (3-fold molar excess) and as coupling reagents N,N,N',N'-tetramethyl-O-(1H-benzotriazol-1-yl)uronium hexafluorophosphate (HBTU) or 2-(7-aza-1H-benzotriazole-1-yl)-1,1,3,3-tetramethyluronium hexafluorophosphate (HATU) (3-fold molar excess) for selected couplings and N,N-diisopropylethylamine (DIEA) (4.5-fold molar excess) in N,N-dimethylformamide (DMF). Cysteines were coupled using 1-hydroxybenzotriazole (HOBt) and diisopropylcarbodiimide (DIC). $N^{\alpha}$-terminal fluorescein-labels were introduced by coupling 5(6)-carboxyfluorescein (3-fold molar excess) using (HATU (3-fold molar excess) and DIEA (4.5-fold molar excess) in DMF (double coupling). In the case of the SPPS of IAPP, pseudoproline dipeptides (3-fold molar excess) were used at specific sequence positions and coupled using HATU (3-fold excess) and DIEA (4.5 fold excess); SPPS, cleavage of protecting groups/from the resin, disulfide bridge formation, and RP-HPLC purification were performed in accordance with standard protocols.

Disulfide bridge formation of the MCIPs and **4Ala-2b** was performed in aq. 0.1 M $NH_4HCO_3$ solution containing DMSO (40%) (stirring for ~1.5 h). SPPS was performed manually for sequence parts containing N-methyl amino acids and either manually or with a CS336X peptide synthesizer (CSBio) for the rest of the sequence parts or peptides containing no N-methyl amino acids. RP-HPLC purifications were preformed using Nucleosil 100 C18 (Grace) or Reprosil Gold 200 C18 columns (Dr. Maisch) according to previously developed standard protocols. Peptide purity was verified by MALDI-TOF mass spectrometry (MS).

Peptide stock solutions were freshly made in 1,1,3,3,3,3-hexafluoro-2-isopropanol (HFIP) on ice; concentrations were determined by peptide weight and/or the BCA assay and by UV spectroscopy in the case of IAPP and fluorescein-labeled peptides in accordance with standard protocols. Stock solutions of IAPP and fluorescein-labeled peptides in HFIP were filtered over 0.2 mm filters just before the determination of their concentration. Peptides were applied from their HFIP stocks following HFIP evaporation with $N_2$ and reconstitution with assay buffer. Recombinant human αSyn was produced as described in (S. T. Kumar, S. Donzelli, A. Chiki, M. M. K. Syed, H. A. Lashuel, Journal of Neurochemistry 2020, 153, 103-119.) or was purchased from Eurogentech (Anaspec) (catalog number AS-55555 with an additional Gly residue at the N-terminus) or from AlexoTech (Catalog Number AS-600-100). αSyn aliquots were prepared in $ddH_2O$, frozen immediately, lyophilized, and freshly reconstituted with assay buffer for each experiment. Of note, freshly made αSyn solutions at the low μM concentration range used in our studies consisted predominantly of non-toxic monomers based on CD spectroscopy, SEC, ThT binding, the MTT reduction assay, and TEM (Fig. 2a, 3c, 3f, 3g, 2a).

### 2. Thioflavin T (ThT) binding assays

Effects of peptides on αSyn self- and cross-seeded amyloid self-assembly were studied by a combination of the ThT binding assay with TEM and the MTT reduction assay according to previously developed protocols (K. Tas, B. D. Volta, C. Lindner, O. El Bounkari, K. Hille, Y. Tian, X. Puig-Bosch, M. Ballmann, S. Hornung, M. Ortner, S. Prem, L. Meier, G. Rammes, M. Haslbeck, C. Weber, R. T. A. Megens, J. Bernhagen, A. Kapurniotu, Nat Commun 2022, 13, 5004).

The ThT binding assays were performed based on a previously published protocol (Shaykhalishahi, A. Gauhar, M. M. Wordehoff, C. S. Gruning, A. N. Klein, O. Bannach, M. Stoldt, D. Willbold, T. Hard, W. Hoyer, Angew Chem Int Ed Engl 2015, 54, 8837-8840; bM. M. Wördehoff, W. Hoyer, Bio-protocol 2018, 8).

Solutions used for the assays of amyloid self-assembly of αSyn alone (3 μM) or its mixtures with various peptide amounts (as indicated) with or w/o preformed (cross-)seeds (10% or 1% as indicated) or related controls were made in 20 mM sodium phosphate buffer (pH 6.0) containing 50 mM NaCl (abbreviated "ThT assay buffer") and ThT (40 μM) in 96-well black MTPs (FluoroNunc/Thermo Fisher Scientific) and incubated ("aged") for up to 7 days at 37°C with shaking at 800 rpm in a MTP shaker (Thermoshaker TPS-4H; 4 More Labor). Of note, each MTP well contained one glass bead (2 mm). ThT fluorescence was measured at 486 nm (excitation at 450 nm) using the Multilabel reader VictorX3 (Perkin Elmer Life Sciences) at the indicated time points with short shaking (1 min) before measuring. In each assay, solutions were prepared in 3 technical replicates (n=3 wells) and the ThT fluorescence signal of the buffer was subtracted from all samples. Data are means (±SD) of 3 independent assays (n=3 wells each) if not stated otherwise.

Preformed seeds of IAPP fibrils (fIAPP) were prepared by incubating IAPP (128 μM) in ThT assay buffer for 24 h (RT; no shaking); fibril formation was verified by TEM (Fig. 2h (inset)). αSyn fibrils (fαSyn) were prepared by incubating αSyn (69 μM in ThT assay buffer in a MTP well containing a glass bead (2 mm) for 7 days (at 37°C & 800 rpm); their formation was verified by TEM (Fig. 2e (inset)). Preformed seeds were sonicated for 1 min prior to their use.

Solutions used for the incubations were made as follows: (1) To study effects of the peptides on (unseeded) αSyn amyloid assembly, freshly made αSyn (3 μM) or αSyn/peptide mixtures (αSyn/peptide molar ratios as indicated) or control solutions in ThT assay buffer containing 40 μM ThT were incubated for 7 days as mentioned above (37°C & 800 rpm). Of note, peptides were preincubated in ThT assay buffer for 6 h prior mixing with αSyn and ThT to ensure complete dissolution. (2) To study effects of the peptides on αSyn amyloid self-assembly (cross-)seeded with preformed fαSyn or fIAPP seeds, freshly made αSyn (3 μM) and the corresponding αSyn/peptide mixtures with or w/o fαSyn or fIAPP (10% (0.3 μM) or 1% (0.03 μM) as indicated; αSyn/peptide molar ratios as indicated), or control solutions (e.g. seeds alone) were incubated in ThT assay buffer containing 40 μM ThT for 7 days as mentioned above (at 37°C & 800 rpm). Of note, peptides with or w/o fαSyn or fIAPP were preincubated in ThT assay buffer (for 6 h, RT) prior to mixing with αSyn and ThT. To investigate the (cross-)seedability of the αSyn/peptide hetero-complexes by fαSyn or fIAPP seeds, peptides (αSyn/-peptide molar ratios as indicated) were preincubated (6 h, RT) with αSyn (3 μM) in ThT assay buffer prior to mixing with fibril seeds to allow for hetero-complex formation. Following addition of fasyn or fLAPP seeds (10% (0.3 μM), solutions were incubated in ThT assay buffer containing 40 μM ThT for 7 days as mentioned above (at 37°C & 800 rpm).

### 3. Assessment of cell damage by the MTT reduction assay

PC12 cells were obtained from DSMZ - German Collection of Microorganisms and Cell Cultures GmbH (DSMZ number: ACC 159) and were cultured and plated as described in (M. Yan, A. Velkova, M. Tatarek-Nossol, E. Andreetto, A.

Kapurniotu, Angew Chem Int Ed Engl 2007, 46, 1246-1252).

Effects of MCIPs and controls on formation of cell-damaging αSyn assemblies were studied by the MTT reduction assay using solutions from the ThT binding assays aged for 0 h, 24 h, 48 h, or 7 days (at 37°C & 800 rpm as under "ThT binding assays") according to previously developed protocols (K. Tas, B. D. Volta, C. Lindner, O. El Bounkari, K. Hille, Y. Tian, X. Puig-Bosch, M. Ballmann, S. Hornung, M. Ortner, S. Prem, L. Meier, G. Rammes, M. Haslbeck, C. Weber, R. T. A. Megens, J. Bernhagen, A. Kapurniotu, Nat Commun 2022, 13, 5004). Briefly, aliquots of ThT binding assay solutions were diluted with cell medium at various incubation time points and added to the PC12 cells at the indicated final concentrations of αSyn and peptides ($IC_{50}$ values at 100 nM αSyn; 7 day-aged solutions). Following incubation with the cells for ~20 h (37°C, humidified atmosphere with 5% $CO_2$), cells were incubated with MTT (0.92 mg/ml) for ~2 h at 37°C. Following addition of 10% SDS in 20 mM HCl (pH 4.5) and overnight incubation, generated formazan was quantified by its absorbance at 570 nm with a Multilabel reader VictorX3 (Perkin Elmer Life Sciences); for 100% MTT reduction the absorbance of untreated cells and for 0% MTT reduction the absorbance of Triton X-100-treated cells was used.

### 4. Transmission electron microscopy (TEM)

Aliquots (10 μl) of solutions to be examined, including solutions used for ThT binding and MTT reduction assays, were applied at the indicated incubation time points on formvar/carbon-coated grids (3 min). Grids were washed with ddH2O and stained with aqueous 2% (w/v) uranyl acetate solution for 1 min. Examination of the grids was performed using a JEOL 1400 Plus electron microscope at 120 kV.

### 5. Far-UV CD spectroscopy

CD measurements were performed using a Jasco 715 spectropolarimeter and spectra (average of 3 spectra) were recorded between 200 and 250 nm, at 0.1 nm intervals, with a response time of 1 sec as previously described in (A. Spanopoulou, L. Heidrich, H. R. Chen, C. Frost, D. Hrle, E. Malideli, K. Hille, A. Grammatikopoulos, J. Bernhagen, M. Zacharias, G. Rammes, A. Kapurniotu, Angew Chem Int Ed Engl 2018, 57, 14503-14508). For the CD studies on structures and effects of αSyn/MCIP hetero-complexes on αSyn self-assembly (Fig. 3c,d), freshly made solutions of αSyn alone (1 μM), MCIP alone (10 μM), and αSyn/MCIP mixtures (1/10) in ThT assay buffer were made and incubated for 48 h at 37° C to mimic the experimental conditions of the ThT binding assays. CD spectra were recorded at the 0 h incubation time point (after a 20 min preincubation at 37° C) and at the 48 h incubation time point. Of note, the spectra of the MCIPs alone are not shown in Fig. 3c,d as they were the same as reported previously in WO2019/234157. The spectrum of the buffer alone was subtracted from all CD spectra.

### 6. Fluorescence spectroscopic titrations

Fluorescence spectroscopic titration studies were performed using a Jasco FP-6500 fluorescence spectrophotometer and previously established protocols (Andreetto, E. Malideli, L. M. Yan, M. Kracklauer, K. Farbiarz, M. Tatarek-Nossol, G. Rammes, E. Prade, T. Neumuller, A. Caporale, A. Spanopoulou, M. Bakou, B. Reif, A. Kapurniotu, Angew Chem Int Ed Engl 2015, 54, 13095-13100). Briefly, excitation was at 492 nm and emission spectra were recorded between 500 and 600 nm. For all experiments, freshly prepared stocks of peptides and their fluorescently labeled analogs in HFIP were used while αSyn stocks were in aqueous 10 mM sodium phosphate buffer (pH 7.4). Measurements were performed in freshly made solutions containing synthetic $N^{\alpha}$-terminal fluorescein-labeled IAPP (Fluos-IAPP), IAPP(8-28) (Fluos-IAPP(8-28)), MCIPs (Fluos-**2e** & Fluos**-2b),** or **4Ala-2b** (Fluos-**4Ala-2b**) (1 or 5 nM as indicated) alone or with various amounts of the binding partner (αSyn, αSyn segments, or IAPP) in aqueous 10 mM sodium phosphate buffer (pH 7.4) containing 0.5% HFIP (for binding to αSyn or its segments) or 1% HFIP (for binding to IAPP or IAPP(8-28)). Measurements were performed within 2-5 min after solution preparation. Under these experimental conditions, Fluos-peptides were mostly monomeric. Apparent (app.) Kd values were calculated as described using 1/1 binding models which fitted the 1/1 αSyn/peptide ratio required for full inhibition of amyloid self-assembly and cytotoxicity. However, due to the self-assembly propensities of the examined peptides/proteins and their multi-site binding features more complex models may also apply. App. Kas are means (±SD) of three binding curves derived from 3 titration assays.

### 7. Cross-linking, NuPAGE, and Western blot (WB) analysis

Cross-linking studies were performed in combination with NuPAGE and WB based on a previously developed assay system (Tas, B. D. Volta, C. Lindner, O. El Bounkari, K. Hille, Y. Tian, X. Puig-Bosch, M. Ballmann, S. Hornung, M. Ortner, S. Prem, L. Meier, G. Rammes, M. Haslbeck, C. Weber, R. T. A. Megens, J. Bernhagen, A. Kapurniotu, Nat Commun 2022, 13, 5004). Briefly, for characterizing αSyn homo- and αSyn/MCIP hetero-assemblies, αSyn alone (10 μM), αSyn/MCIP mixtures (1/5), and MCIPs alone (50 μM) were prepared in ThT assay buffer. Following incubation for 30

min, solutions were cross-linked by adding 25% aqueous glutaraldehyde (Sigma-Aldrich) (2 min). Following treatment with 2 M $NaBH_4$ (in 0.1 M NaOH, 20 min), cross-linked complexes were precipitated with 10% aqueous trichloroacetic acid (4°C), centrifuged (10 min, 12000 g), and pellets were dissolved in NuPAGE LDS-sample buffer (w/o reducing agent) boiled for 5 min, and subjected to gel electrophoresis (10-20% Tricine gels) with Tricine SDS running buffer according to the manufacturer's (Invitrogen) recommendations. Equal amounts of aSyn (4.3 μg) and MCIPs (2.5 μg for **2b** and **2e** and 2.2 μg for **4Ala-2b)** were loaded in the different lanes and prestained protein size markers (from 3.5 to 260 kDa (Invitrogen)) were run in the same gels.

Gels were blotted onto nitrocellulose membranes using an XCell II Blot Module blotting system (Invitrogen) and membranes were blocked by overnight incubation (10°C) with 2% BSA in TBS-T (20 mM Tris, 150 mM NaCl, 0.05% Tween-20). To detect αSyn containing bands, membranes were incubated with a rabbit anti-human αSyn antibody (Sigma-Aldrich, SAB4502829, Lot: 210582) (1:1000) in 0.5% BSA in TBS-T (2 h at RT or overnight at 10°C) and thereafter with donkey anti-rabbit-HRP antibody (GE Healthcare, NA934, Lot: 16836138) (1:5000) in 0.5% BSA in TBS-T (2 h at RT or overnight at 10°C). Western blots were developed using Super Signal West Dura Extended Duration Substrate (Thermo Scientific, Cat. No. 34075) and imaged using a LAS-4000 mini imager (Fujifilm). To detect **2e-** or **2b**-containing bands, membranes were stripped by incubating with stripping buffer consisting of 2% SDS and 100 mM β-mercaptoethanol in 50 mM Tris (pH 6.8) (40 min at 60°C and 60 min at RT). After washing with TBS-T, membranes were blocked with 2% BSA in TBS-T (3 h, RT). Detection of **2b** and **2e** was done by incubation (2 h at RT or overnight at 10°C) with mouse anti-**2e** antibody (Clone MCP2E 26C3), which was produced at Helmholtz Center Munich as described below, and with goat anti-mouse-HRP antibody (Abcam, ab6789, Lot: GR3257574-1) (1:10000) in 0.5% BSA in TBS-T as secondary antibody (2 h at RT or overnight at 10°C). Of note, the anti-**2e** antibody recognized **2b** as well and did not bind with αSyn (Fig. 3e); the anti-αSyn antibody did not bind to **2b** and **2e** (data not shown).

### 8. Generation of monoclonal antibodies against 2e

Monoclonal antibodies were developed in cooperation with the core facility monoclonal antibodies of the Helmholtz Center Munich as follows: Thirteen week old female Balb/c wild type mice (Charles River) were immunized subcutaneously (s.c.) and intraperitoneally (i.p.) with a mixture of 50 μg ovalbumin-coupled 2e dissolved in a mixture of 200 μl PBS containing 5 nmol CpG2006 (TIB MOLBIOL) and 200 μL incomplete Freund's adjuvant (Sigma-Aldrich). Animal procedures were approved by the local Animal Use and Care Committee with approval by the local authorities of Upper Bavaria, Germany (reference number ROB-55.2Vet-2532.Vet_03-22-25) in accordance with European and German animal welfare regulations. After 12 weeks, a boost without Freund's adjuvant was given i.p. and s.c. 3 days before fusion. Fusion of the myeloma cell line P3X63-Ag8.653 with the mouse immune spleen cells was performed using polyethylene glycol 1500 according to standard procedure. After fusion, the cells were plated in 96-well plates with medium consisting of RPMI 1640 with stable glutamine (RPMI-STA, Capricorn) supplemented with 20% fetal calf serum, 1mM pyruvate, 1x non-essential amino acids and HAT media supplement (Hybri-Max, Sigma-Aldrich). Hybridoma supernatants were screened 10 days later in a flow cytometry assay (iQue, Intellicyt; Sartorius) using N-terminal biotinylated **2e** captured on streptavidin beads (PolyAN) and incubated for 90 min with hybridoma supernatant and Atto-488-coupled isotype-specific monoclonal rat-anti-mouse IgG secondary antibodies. Antibody binding was analyzed using ForeCyt software (Sartorius). Positive supernatants were further validated by dot blot and Western blot. Hybridoma cells from selected supernatants were cloned by limiting dilution to obtain stable monoclonal cell lines. Our assays (Fig. 3e) were performed with hybridoma supernatant of clone MCP2E 26C3 (mouse IgG2a/κ).

### 9. Size exclusion chromatography (SEC) and ESI-MS of collected peaks

For the SEC studies a Superdex 75 10/300 GL column (GE Healthcare) and a Dionex UltiMate 3000 device (Thermo Fisher Scientific) were used (Tas, B. D. Volta, C. Lindner, O. El Bounkari, K. Hille, Y. Tian, X. Puig-Bosch, M. Ballmann, S. Hornung, M. Ortner, S. Prem, L. Meier, G. Rammes, M. Haslbeck, C. Weber, R. T. A. Megens, J. Bernhagen, A. Kapurniotu, Nat Commun 2022, 13, 5004). Elution buffer was the ThT assay buffer, the flow rate was 0.5 ml/min, and protein/peptide detection was at 214 nm. The column was calibrated with a gel filtration protein standard (Bio-Rad, Cat. No. 151-1901) (Supporting Fig. S10). The retention time ($t_R$) of αSyn monomers was determined by injecting αSyn (3 μM) freshly dissolved in 6 M GdnHCl in 0.1 M $NH_4HCO_3$ (pH 8.4). Of note, αSyn monomers eluted earlier than expected based on the applied globular protein standard likely due to their disordered structure consistent with previous reports. For the analysis of αSyn/MCIP hetero-complexes, freshly made solutions (400 μl) of αSyn alone (3 μM), **2e** alone (30 μM), and αSyn/**2e** mixtures (1/10) in ThT assay buffer were loaded onto the column. Peaks were collected, lyophilized, and analyzed by liquid-chromatography electrospray ionization mass spectrometry (LC-ESI-MS). For sample analysis, a Dionex UltiMate 3000 HPLC system (column: MSPac DS-10 (10 × 2.1 mm, 5 μm) (Thermo Scientific)) coupled to a LCQ Fleet mass spectrometer (Thermo Scientific) with a heated ESI source was used. The mass spectrometer was run in positive

mode collecting ion-trap scans at scan rate "normal" from 300 to 2000 m/z. Spectra were visualized using XCalibur 2.2 SP1.48 (Thermo Scientific) and deconvoluted using MagTran 1.02 (Amgen Inc.) implementing the ZSCORE algorithm (Z. Zhang, A. G. Marshall, Journal of the American Society for Mass Spectrometry 1998, 9, 225-233).

## 10. Slot blot analysis

Effects of MCIPs on kinetics of formation of cytotoxic (A11-reactive) αSyn oligomers (Fig. 3g) were studied by Slot blot analysis in combination with ThT binding, MTT reduction assays, and TEM (see Fig. 2a-c). Slot blot analysis was performed using a PR648 Slot blot blotting manifold (Hoefer) with nitrocellulose membrane based on a previously described protocol (C. Kontos, O. El Bounkari, C. Krammer, D. Sinitski, K. Hille, C. Zan, G. Yan, S. Wang, Y. Gao, M. Brandhofer, R. T. A. Megens, A. Hoffmann, J. Pauli, Y. Asare, S. Gerra, P. Bourilhon, L. Leng, H. H. Eckstein, W. E. Kempf, J. Pelisek, O. Gokce, L. Maegdefessel, R. Bucala, M. Dichgans, C. Weber, A. Kapurniotu, J. Bernhagen, Nat Commun 2020, 11, 5981). Briefly, solutions containing αSyn (3 μM) alone, **2e** (3 μM) alone, and their mixture (1/1) in ThT assay buffer were incubated for 7 days (37°C, 800 rpm) in MTPs as for the ThT binding assay and added (200 μl each) at the indicated incubation time points to the slots which had been pre-wetted with ThT assay buffer. Solutions were left in the slots for 2 h (RT) to allow for binding with the membrane; thereafter, they were removed with vacuum and slots were washed with ThT assay buffer (1×100 μL). The membrane was washed 3×5 min with TBS-T and blocked with 2% BSA in TBS-T overnight at 10°C. Then, the membrane was incubated with a rabbit A11 antibody (Invitrogen, AHB0052, Lot: WH329538) (1:1000 in 0.5% BSA in TBS-T) (overnight at 10°C) and, following washing with TBS-T (3×5 min), incubated with donkey anti-rabbit-HRP antibody (GE Healthcare, NA934, Lot: 16836138) (1:5000 in 0.5% BSA in TBS-T) 2h at RT or overnight at 10°C. Following washing with TBS-T (3×5 min), the membrane was developed using Super Signal West Dura Extended Duration Substrate (Thermo Scientific, Cat. No. 34075) and imaged with a LAS-4000 mini imager (Fujifilm).

## 11. Dot blot assays

Binding of **2b, 2e** and **4Ala-2b** to fIAPP or fαSyn (Fig. 3h) was studied by dot blot analysis performed according to a previously described protocol (Tas, B. D. Volta, C. Lindner, O. El Bounkari, K. Hille, Y. Tian, X. Puig-Bosch, M. Ballmann, S. Hornung, M. Ortner, S. Prem, L. Meier, G. Rammes, M. Haslbeck, C. Weber, R. T. A. Megens, J. Bernhagen, A. Kapurniotu, Nat Commun 2022, 13, 5004). Briefly, fIAPP was prepared by incubating an IAPP solution (128 μM) in 50 mM sodium phosphate buffer, pH 7.4, containing 100 mM NaCl and 0.5% HFIP for 24 h at RT (fIAPP confirmed by ThT and TEM) (Tas, B. D. Volta, C. Lindner, O. El Bounkari, K. Hille, Y. Tian, X. Puig-Bosch, M. Ballmann, S. Hornung, M. Ortner, S. Prem, L. Meier, G. Rammes, M. Haslbeck, C. Weber, R. T. A. Megens, J. Bernhagen, A. Kapurniotu, Nat Commun 2022, 13, 5004). fαSyn were prepared by incubating αSyn (69 μM) in ThT assay buffer at 37°C & 800 rpm for 7 days as for the ThT binding assay (confirmed by ThT and TEM; data not shown). fIAPP (20 μg) or fαSyn (10 μg) were spotted on nitrocellulose membranes. Membranes were washed with TBS-T, blocked with 5% milk in TBS-T overnight at 10°C, and washed again with TBS-T. Then, membranes were incubated with solutions of N-terminal fluorescein-labeled peptides (Fluos-peptide) (1.5 μM) in 50 mM sodium phosphate buffer, pH 7.4, containing 100 mM NaCl and 0.5% HFIP (for fIAPP binding studies) or in ThT assay buffer (for fαSyn binding studies) overnight at 10°C. To control for fibril autofluorescence, membranes containing spotted fIAPP or fαSyn were incubated in parallel with buffer alone (Tas, B. D. Volta, C. Lindner, O. El Bounkari, K. Hille, Y. Tian, X. Puig-Bosch, M. Ballmann, S. Hornung, M. Ortner, S. Prem, L. Meier, G. Rammes, M. Haslbeck, C. Weber, R. T. A. Megens, J. Bernhagen, A. Kapurniotu, Nat Commun 2022, 13, 5004). Following washing with TBS-T, bound peptides were visualized with a LAS-4000 mini imager equipped with a suitable fluorescence filter (Fujifilm).

## 12. Studies using peptide arrays

To identify the αSyn binding sites for its interactions with MCIPs and IAPP, we used synthetic peptide arrays. Peptide arrays containing IAPP or αSyn decamers covering full length IAPP or αSyn and positionally shifted by one residue were synthesized on a modified cellulose membrane support using stepwise SPOT synthesis protocols and a MultiPep RSi (Intavis) peptide synthesizer as previously described (Andreetto, L. M. Yan, M. Tatarek-Nossol, A. Velkova, R. Frank, A. Kapurniotu, Angew Chem Int Ed Engl 2010, 49, 3081-3085). Thereafter, arrays were immobilized on glass slides according to the manufacturer's instructions and processed and developed using previously described protocols (Andreetto, L. M. et al., 2010, ibid.). Briefly, glass slides blocked for 4 h at RT with 1% BSA in TBS-T and incubated with synthetic $N^{\alpha}$-terminal fluorescein-labeled **2e** (Fluos-**2e**) or IAPP (Fluos-IAPP) (1 μM in 1% BSA in TBS-T or with recombinant Biotin-labeled M1C-αSyn (Biotin-αSyn; Cys1(side-chain)-labeled M1C-αSyn via thiol-maleimide-chemistry) (0.5 μM in 1% BSA in TBS-T) overnight (at 10°C) followed by washing with TBS-T. Bound peptides were visualized by using a LAS-4000 mini instrument as follows (Fujifilm): In the case of fluorescein-labeled peptides, visualization was based on their fluorescence readout. In the case of Biotin-αSyn, visualization was performed via enhanced chemiluminescence (ECL) following incubation (2h at RT) of the glass slides with streptavidin-POD antibody (Roche Diagnostics,

11089153001, Lot: 56790500) (1:1000 in 1% BSA in TBS-T) and development by the SuperSignal West Dura Extended Duration Substrate (Thermo Scientific, Cat. No. 34075) as described (Andreetto, L. M. et al., 2010, ibid.). For quantification, the optical density of each spot was determined using ImageJ software. For each array, the highest optical density value was defined as 100%, while the optical density value of the negative control spot was defined as 0% and relative intensity of each spot was determined by using the following formula:

$$Rel.\,intensity = \frac{optical\ density\ (spot) - optical\ density\ (neg.\,control, 0\%)}{highest\ optical\ density\ (100\%) - optical\ density\ (neg.\,control, 0\%)}$$

Binding regions corresponded to clusters of at least 4 strong binding decamers defined as indicated in the legends of Supporting Fig. S16 and S18. Of note, results of densitometric analyses were consistent with the results of the visual inspection of the arrays.

**13. Fluorescence polarization assays**

Fluorescence polarization (FP) assays were performed using a Jasco FP-8550 fluorescence spectrophotometer. Excitation was at 492 nm and emission was recorded at 522 nm. Bandwidth for excitation and emission was set at 5 nm and time response at 0.5 s. Measurements were performed within 2-5 min upon solution preparation. To study interactions of the three identified αSyn key segments αSyn(1-14), αSyn(34-52), and αSyn(87-105) with fIAPP, we measured FP of freshly made Fluos-fIAPP alone (100 nM) in aqueous 10 mM sodium phosphate buffer (pH 7.4) containing 0.5% HFIP and its mixtures with each of the 3 segments (1.5 μM). Fluos-fIAPP was made by incubating Fluos-IAPP (20 μM) in aqueous 10 mM sodium phosphate buffer (pH 7.4) containing 0.5% HFIP for 48 h; solutions were sonicated for 30 seconds prior to their use for FP assays; the presence of fibrils was verified by TEM (not shown).

**14. Preparation and characterization of αSyn oligomers for the ex-vivo hippocampal LTP measurements**

αSyn oligomers for the hippocampal LTP measurements were prepared based on a previously reported protocol (M. J. Diogenes, R. B. Dias, D. M. Rombo, H. Vicente Miranda, F. Maiolino, P. Guerreiro, T. Nasstrom, H. G. Franquelim, L. M. Oliveira, M. A. Castanho, L. Lannfelt, J. Bergstrom, M. Ingelsson, A. Quintas, A. M. Sebastiao, L. V. Lopes, T. F. Outeiro, Journal of Neuroscience 2012, 32, 11750-11762). Briefly, lyophilized αSyn was suspended at 2 mg/ml (138 μM) in dd$H_2O$ and incubated under continuous shaking for 5 days at 37°C in a Thermomixer (Eppendorf) at 1400 rpm. Solutions were centrifuged at 22000g for 15 min, the resulting supernatant (oligomers) was lyophilized. The concentration of αSyn oligomer solutions (supernatants) was determined using the BCA assay (Pierce). Supernatant fractions containing the oligomers were lyophilized, kept at -60°C, and reconstituted with aCSFjust prior to the LTP measurements. In addition, assemblies present in supernatants and pellets of the αSyn oligomer preparations were characterized by TEM, the ThT binding assay, the MTT reduction assay, and a Dot blot assay to assess An-reactivity using above protocols (). Briefly, TEM grids were prepared and imaged as described under TEM. ThT binding was determined in aliquots of solutions containing αSyn (1.7 μM) monomers (freshly made and supernatant (oligomer) or pellet (fibril) fractions in ThT assay buffer containing 20 μM ThT as described under "ThT binding assays". For determination of A11-reactivity, solutions containing mostly αSyn monomers and the supernatants or re-suspended pellets (69 μM in dd$H_2O$) were spotted on a nitrocellulose membrane (as described under dot blot assays) and the membrane was blocked with 2% BSA in TBS-T overnight at 10°C and incubated with rabbit A11 antibody and developed as described under "Slot blot assays". For the assessment of the cell-damaging effects of αSyn oligomer-containing fractions, lyophilized supernatant fractions were dissolved in ThT assay buffer to a concentration of 3 μM (as for the ThT binding assays). Following dilution with cell medium, αSyn oligomers were added to the cultured PC12 cells plated in MTPs (aSyn, 300 nM) and incubated with the PC12 cells for ~20 h as described under "MTT reduction assays". MTT reduction was assessed as described under "MTT reduction assays". Data is means (±SD) of four assays (n=3 wells each) performed using three independent αSyn oligomer preparations.

**15. Hippocampal long-term potentiation (LTP) measurements (ex vivo)**

LTP measurements were performed as previously described (K. Tas, B. D. Volta, C. Lindner, O. El Bounkari, K. Hille, Y. Tian, X. Puig-Bosch, M. Ballmann, S. Hornung, M. Ortner, S. Prem, L. Meier, G. Rammes, M. Haslbeck, C. Weber, R. T. A. Megens, J. Bernhagen, A. Kapurniotu, Nat Commun 2022, 13, 5004). Briefly, sagittal hippocampal slices (350 μm) were obtained from C57BL/6N mice (6-8 weeks of age, male) (Charles River Laboratories) in ice-cold Ringer solution bubbled with a mixture of 95% $O_2$ and 5% $CO_2$ according to protocols approved by the ethical committee on animal care and use of the government of Bavaria Germany (according to §11 TierschG and §4 TierschG; no accreditation number). Ethics oversight by ethical committee on animal care and use of the government of Bavaria (Regierung von Oberbayern,

ROB). Extracellular recordings were performed using artificial cerebrospinal fluid (aCSF)-filled glass microelectrodes (2-3 MΩ) at RT. aCSF consisted of 125 mM NaCl, 2.5 mM KCl, 25 mM $NaHCO_3$, 2 mM $CaCl_2$, 1 mM $MgCl_2$, 25 mM D-glucose, and 1.25 mM $NaH_2PO_4$ (pH 7.3) and was bubbled with 95% $O_2$ and 5% $CO_2$. Field excitatory postsynaptic potentials (fEPSPs) were evoked in the hippocampal CA1 dendritic region via two independent inputs by stimulating the Schaffer collateral commissural pathway (Seep). For LTP induction, high-frequency stimulation (HFS; 100Hz/100 pulses) conditioning pulses were delivered to the same Seep inputs. Both stimulating electrodes were used to utilize the input specificity of LTP, thus allowing for the measurement of internal control within the same slice. αSyn oligomers (175 nM) (made as described in the previous chapter), their mixtures with MCIPs (1/10), or MCIPs alone (1.75 μM) were freshly dissolved in aCSF and applied to the slices 60-90 min before HFS. Responses were measured for 60 min after HFS. fEPSP slope measurements (20-80% of peak amplitude) are presented as %fEPSP slope of baseline (the 20 min control period before tetanic stimulation was set to 100%). For statistical comparisons, we performed non-parametric testing with Mann-Whitney U tests or a Kruskal-Wallis test. A correction for multiple comparisons was not performed, which is acceptable as for instance stated by Rothman (K. J. Rothman, Epidemiology 1990, 1, 43-46) and uncorrected p-values are presented in Fig. 4.

**16. Sequences**

The key amyloid protein in Parkinson's disease (PD) is "a-synuclein" (αSyn) which has an amino acid sequence of 140 residues as follows:

```
MDVFMKGLSK AKEGVVAAAE KTKQGVAEAA GKTKEGVLYV GSKTKEGVVH
GVATVAEKTK EQVTNVGGAV VTGVTAVAQK TVEGAGSIAA ATGFVKKDQL
GKNEEGAPQE GILEDMPVDP DNEAYEMPSE EGYQDYEPEA
```

The sequence of the key amyloid protein in diabetes type 2 is islet amyloid polypeptide (IAPP) which has the following 37 residue amino acid sequence:
KCNTATCATQ RLANFLVHSSN NFGAILSSTN VGSNTY

R3-GI, an IAPP interaction interaction surface mimic (or "ISM") is a linear peptide which has the sequence:
ATQRLANFLV HRRRNFGAIL S
in which G17 and I19 (= G24 and I26, when using an IAPP-based numbering) are N-methylated (Andreetto, Kapurniotu et al. ACIE (2015)).

The negative control **"4Ala-2b"** (Spanopoulou, Kapurniotu et al. ACIE (2018)) in which all four IAPP-derived key residues (for IAPP self/cross-interactions) of **2b** and **2e** (F15, L16, F23 and I26 of the IAPP-sequence) were replaced by Ala, has a cyclized sequence of
CGAAGGRRRG AGGAGGC
disulfide cyclization occurring via the terminal cysteines, with G12 and A14 additionally being N-methylated.

## Example 2

### Nanomolar Affinity IAPP/αSyn Cross-Interactions Mediated by IAPP Amyloid Core Region IAPP(8-28)

The inventors first determined the IAPP regions that mediate its cross-interactions with αSyn. Synthetic peptide arrays containing IAPP decamers covering full-length IAPP and positionally shifted by one residue were incubated with biotin-labeled αSyn (Biotin-αSyn) and Biotin-αSyn-bound decamers were visualized by chemiluminescence. The inventors found a major cluster of 4 consecutive decamers within IAPP(8-20), while a second weaker cluster localized in IAPP(13-27) (Fig. 1a). The inventors then titrated synthetic $N^{\alpha}$-terminal fluorescein-labeled IAPP (Fluos-IAPP) and IAPP(8-28) (Fluos-IAPP(8-28)) with αSyn. Determined app. Kis were 26.7 (±6.0) nM for the Fluos-IAPP/αSyn interaction and 8.2 (±2.3) nM for the Fluos-IAPP(8-28)/αSyn interaction (Figure 1b,c). These data revealed that IAPP binds αSyn with low nanomolar affinity and that the IAPP amyloid core IAPP(8-28) contains the key recognition elements for the IAPP/αSyn interaction as earlier found (WO2019/234157 A1) for the IAPP/IAPP and the IAPP/Aβ40(42) interactions.

### Macrocyclic peptides 2b and 2e are Nanomolar Inhibitors of Self- and IAPP-Cross-Seeded Amyloid Self-Assembly of αSyn

Based on the above, the inventors hypothesized that the IAPP(8-28)-derived macrocyclic peptides as described earlier in WO2O19/234157 of formulae 0, 0a, 1, 1*, 2a - 2e, 2a* - 2e* might mimic putative IAPP/αSyn cross-interaction surfaces and interfere with αSyn amyloid self-assembly and its cross-seeding by fIAPP. To test this they used peptides of

formulae **2b** and **2e** as examples. Notably, initial studies showed that both IAPP(8-28), which is intrinsically amyloidogenic, and its non-amyloidogenic analogs IAPP(8-28)-GI and R3-GI, which are linear MCIP precursors, were unable to inhibit (data not shown). Hence, in the following, peptides 2b and 2e were used as exemplary test peptides. However, the present inventors believe that all the other peptides of formulae 0, 0a, 1, 1*, 2a - 2e, 2a* - 2e*, as described herein, behave similarly and show the same inhibitory effects, as demonstrated herein.

The effects of **2b** and **2e** on αSyn amyloid self-assembly were then studied (Fig. 2, Table 1). In parallel, the inventors also studied the effects of the negative control peptide **4Ala-2b.** According to the amyloid specific ThT binding assay and transmission electron microscopy (TEM), αSyn fibrillogenesis started after a lag-time of ~24 h and was accomplished after ~48-72 h (Fig. 2a,b). However, in the presence of **2b** or **2e** (αSyn/peptide 1/1) a full suppression of αSyn fibrillogenesis was observed (Figures 2a,b). In addition, **2b** and **2e** strongly suppressed formation of cell-damaging αSyn assemblies according to the results of the 3-[4,5-dimethylthiazol-2-yl]-2,5-diphenyltetrazolium bromide (MTT) reduction assay in cultured rat pheochromocytoma (PC12) cells (Fig. 2c). In fact, αSyn titrations with **2b** and **2e** revealed nanomolar $IC_{50}$ values, i.e. 62.3 (±33.9) nM (**2b**) and 66.0 (±23.3) nM (**2e**) (Table 1). No attenuating effects were found for the negative control **4Ala-2b** up to a 50-fold higher molar excess than **2b** or **2e** (Fig. 2a-c).

**Table 1.** $IC_{50}$ of inhibitory effects of **2b, 2e,** and **4Ala-2b** on cell-damaging effects of unseeded, fαSyn-seeded, and fIAPP-cross-seeded αSyn amyloid self-assembly.

| Peptide | $IC_{50}$ (±SD) (nM) Inhibition of αSyn[a] | $IC_{50}$ (±SD) (nM) Inhibition of αSyn + fαSyn[a] | $IC_{50}$ (±SD) (nM) Inhibition of αSyn + fIAPP[a] |
|---|---|---|---|
| **2b** | 62.3 (±33.9) | 51.8 (±4.8) | 75.2 (±21.1) |
| **2e** | 66.0 (±23.3) | 54.7 (±4.4) | 42.0 (±18.5) |
| **4Ala-2b** | > 5000 | > 5000 | > 5000 |

[a] $IC_{50}$ values, means (±SD) from 3 titration assays (n=3 wells each); αSyn, 100 nM w/o or with preformed fαSyn or fIAPP seeds (10%).

The inventors next asked whether **2b** and **2e** might also suppress seeding of αSyn fibrillogenesis by preformed αSyn fibrils (fαSyn). Addition of fαSyn seeds (10%) to αSyn strongly accelerated formation of αSyn fibrils and cell-damaging aggregates as expected (Fig. 2d-f). However, in the presence of **2b** and **2e** (1/1), αSyn fibrillogenesis and cell toxicity were fully suppressed whereas again **4Ala-2b** (αSyn/**4Ala-2b,** 1/50) did not inhibit (Fig. 2d-f). Titrations with the two inhibitors yielded $IC_{50}$ values of 51.8 (±4.8) nM (**2b**) and 54.7 (±4.4) nM (**2e**) which were nearly identical to the $IC_{50}$ values of effects on unseeded αSyn fibrillogenesis (Table 1). Notably, **2b** and **2e** inhibited αSyn fibrillogenesis and cytotoxicity when seeding was performed both with 10% and 1% fαSyn seeds, indicative of effects on secondary nucleation and fibril elongation events (Fig. 2d-f).

The inventors then asked whether the two peptides might also interfere with the cross-seeding effect of IAPP fibrils (fIAPP) on αSyn fibrillogenesis. Addition of seed amounts (10%) of preformed IAPP fibrils (fIAPP) to αSyn strongly accelerated its fibrillogenesis consistent with previous findings (Fig. 2g,h). In parallel, a strong acceleration of formation of cell-damaging αSyn species was also observed (data not shown). Importantly, in the presence of **2b** or **2e** (1/1) a full suppression of cross-seeding of αSyn fibrillogenesis and cytotoxicity was observed and titrations yielded nanomolar $IC_{50}$ values for both peptides, i.e. 75.2 (±21.1) nM (**2b**) and 42.0 (±18.5) nM (**2e**) (Table 1). As expected, **4Ala-2b** did not inhibit (αSyn/peptide, 1/50).

Taken together, the above studies identified **2b** and **2e** as nanomolar inhibitors of both self- and fIAPP-cross-seeded amyloid self-assembly of αSyn.

**MCIPs Bind αSyn with Nanomolar Affinity and Sequester it into Non-Fibrillar and Non-Cytotoxic Co-Assemblies**

To learn more about the inhibition mechanism, αSyn/peptide interactions and co-assemblies were studied by various biophysical and biochemical methods. First, the affinities of αSyn/peptide interactions were determined by titrating synthetic N-terminal fluorescein-labeled **2b** (Fluos-**2b**) and **2e** (Fluos-**2e**) with αSyn (Fig. 3a,b). Low nanomolar apparent (app.) $K_d$ values were obtained for both peptides (Fluos-**2b**, app. $K_d$=17.2 (±2.6) nM; Fluos-**2e**, 22.0 (±5.1) nM) (Table 2) in good agreement with their $IC_{50}$ values (Table 1). Notably, the αSyn binding affinities of **2b** and **2e** were very similar to their IAPP binding affinities while **4Ala-2b** did not bind either IAPP or αSyn (Table 2).

The far-UV CD spectrum of freshly dissolved αSyn exhibited a pronounced minimum at ~200 nm indicative of mainly disordered structure consistent with previous reports (Fig. 3c,d). Following aging for 48 h, a marked reduction of the CD magnitude was observed indicative of αSyn oligomerization. In the presence of **2b** or **2e**, however, no/slower reduction of the CD magnitude was observed in line with their inhibitory activity on αSyn amyloid self-assembly (Fig. 3c,d). Of note, in

addition to the minimum at ~200 nm, the CD spectra of αSyn/inhibitor mixtures exhibited a weaker but clear minimum between 220-230 nm. Their shapes and magnitudes suggested that hetero-complexes were more ordered than αSyn (Fig. 3c,d).

Next, αSyn/inhibitor hetero-complexes were cross-linked with glutaraldehyde and following separation by NuPAGE visualized by Western blot (WB) with anti-αSyn and anti-**2e**(**2b**) antibodies (Fig. 3e). In freshly made αSyn solutions, monomers and dimers were major species; trimers and other medium-to-high MW aggregates were less abundant consistent with previous findings. In αSyn/**2b**(**2e**) mixtures, a similar pattern as in αSyn alone was observed with the difference that the bands stained with both the anti-αSyn and a monoclonal anti-**2e**(**2b**) antibody; in addition, bands corresponding to αSyn mono-, di-, and trimers were slightly shifted upwards (Fig. 3e). These data indicated that **2b** and **2e** co-assemble with αSyn monomers and low MW oligomers into hetero-dimers and low MW hetero-oligomers (Fig. 3e).

Hetero-complexes formed at early steps of αSyn/2e co-assembly were then studied by size exclusion chromatography (SEC) (Fig. 3f). αSyn monomers (~15 kDa) present in freshly made αSyn alone solutions eluted at a retention time ($t_R$) of ~21 min corresponding to a globular protein of ~44 kDa; this was due to its natively unfolded nature resulting in a higher hydrodynamic radius (Fig. 3f). In **2e** alone (~2 kDa) solutions, the major fraction eluted at ~38 min and corresponded to **2e** monomers while a smaller fraction corresponding to **2e** oligomers eluted at ~32 min. Importantly, in the αSyn/**2e** mixtures, the **21** min peak found in αSyn alone was still present but the **2e** alone peaks were strongly diminished (Fig. 3f). These findings were consistent with formation of αSyn/**2e** hetero-complexes which eluted at -21 min and were confirmed by electrospray ionization mass spectrometry (ESI-MS) (Fig. 3f). The observed lack of a shift of the αSyn peak to higher MWs in the αSyn/**2e** mixture was most likely due to the low MW of **2e** and the resolution limit of the column. Together, the above studies suggested αSyn/**2e** hetero-dimers and low MW hetero-oligomers as early species in the αSyn/**2e** co-assembly pathway.

Formation of cytotoxic αSyn oligomers is associated with neurodegeneration and PD pathogenesis. Our ThT binding and MTT reduction assays suggested that in the presence of the MCIPs formation of cytotoxic assemblies of αSyn was strongly suppressed (Fig. 2a-c). To characterize the effects of MCIPs on formation of αSyn oligomers more directly, kinetics of cytotoxic oligomer formation in αSyn alone and its mixtures with **2e** were followed. The inventors used slot blot analysis and the antibody A11 reported to recognize toxic oligomers of various different proteins including αSyn (Fig. 3g). Formation of cytotoxic αSyn oligomers was confirmed by MTT reduction and TEM (data not shown). In αSyn alone, large amounts of cytotoxic An-reactive oligomers were present in ~48 h-aged solutions (Fig. 3g). By contrast, no An-reactive oligomers and no cytotoxic effects were observed in the αSyn/**2e** mixtures (1/1) (Fig. 3g, Fig. 2c).

The potent inhibitory activity of the MCIPs could also be mediated by binding to fαSyn and/or fIAPP resulting in suppression of secondary nucleation and/or fibril elongation. In fact, dot blot (DB) assays showed that Fluos-**2b** and Fluos-**2e** are able to bind both fαSyn and fIAPP (Fig. 3h). However, the non-inhibitor Fluos-**4Ala-2b** also bound -most likely non-specifically- (Fig. 3h). In addition, sub-stoichiometric amounts of **2b** and **2e** did not markedly affect self-/cross-seeded αSyn fibrillogenesis (data not shown). Furthermore, αSyn/**2e**(**2b**) hetero-complexes were unable to become (cross-) seeded by fαSyn or fIAPP consistent with a key role in MCIPs' anti-amyloid function (data not shown).

Collectively, the findings suggested that the inhibitory effects of **2b** and **2e** are mainly mediated by nanomolar affinity binding to αSyn monomers and/or prefibrillar species and their sequestration into amorphous, non-cytotoxic, and non-(cross-)seedable αSyn/MCIP co-assemblies.

**Table 2**. App. Kas of interactions of Fluos-**2b**, -**2e**, and -IAPP with IAPP, αSyn, and the three identified αSyn key segments determined by fluorescence spectroscopic titrations.[a]

| Binding partner | app. $K_d$ (±SD) (nM) (2b) | app. $K_d$ (±SD) (nM) (2e) | pp. $K_d$ (±SD) (nM) (IAPP) |
|---|---|---|---|
| **IAPP** | 29.6 (±19.3) | 46.9 (±33.4) | 9.7 (±0.9) |
| **αSyn** | 17.2 (±2.6) | 22.0 (±5.1) | 26.7 (±6.0) |
| **αSyn(1-14)** | 366.2 (±115.8) | 461.3 (±47.5) | 886.4 (±552.0) |
| **αSyn(34-52)** | 662.8 (±9.4) | 504.7 (±171.2) | 347.5 (±103.2) |
| **αSyn(87-105)** | 72.1 (±20.5) | 122.2 (±17.1) | 31.9 (±0.6) |

[a] App. Kas, means (±SD) from 3 binding curves using $N^{\alpha}$-terminal fluorescein-labeled **2b** (Fluos-**2b**), **2e** (Fluos-**2e**), and IAPP (Fluos-IAPP) (pH 7.4). Fluos-peptides 5 nM except for titrations with αSyn and of Fluos-IAPP with IAPP (data from Yan et al. PNAS (2006)) (Fluos-peptides 1 nM).

### MCIPs Ameliorate αSyn Oligomer-Mediated Synaptic Damage in Mouse Brains *ex vivo*

The impairment of hippocampal synaptic long term potentiation (LTP) by αSyn oligomers is believed to be directly linked to neuronal dysfunction in PD. Therefore, to obtain first information about the potential physiological relevance of the *in vitro* findings, the inventors investigated the effects of the two MCIPs on αSyn oligomer-mediated impairment of

hippocampal synaptic LTP in mouse brains *ex vivo* (Fig. 4). In fact, the electrophysiological studies showed that synaptic LTP damage caused by preformed cytotoxic αSyn oligomers was significantly reduced in the presence of **2b** or **2e** (Fig. 4).

### Three αSyn Key Regions Mediate its High Affinity Interactions with both the MCIPs and IAPP: Multi-Site Binding Underlies MCIP Anti-Amyloid Function

To identify the αSyn regions mediating its high affinity interactions with the MCIPs, the inventors incubated synthetic peptide arrays containing αSyn decamers covering its entire sequence and positionally shifted by one residue with Fluos-**2e** (Fig. 5a). The inventors identified 3 clusters of strong binding decamers: one localized within the N-terminal segment αSyn(1-14), a 2$^{nd}$ one within αSyn(34-52), and a 3$^{rd}$ one within αSyn(87-105) (Fig. 5a). The results of the peptide array studies were confirmed and quantified by fluorescence spectroscopic titrations which revealed nanomolar app. Kas for the interactions of **2e** and **2b** with all 3 αSyn segments (Fig. 5b-d Table 2). These data showed that the high affinity binding of MCIPs to αSyn is mediated via the 3 αSyn regions αSyn(1-14), αSyn(34-52), and αSyn(87-105).

Because MCIPs might mimic IAPP sites mediating its cross-interactions with αSyn, the inventors hypothesized that they might interact with the same/similar αSyn regions as IAPP which could underlie their potent inhibitory activity on IAPP-mediated cross-seeding. To address this, the αSyn peptide array was incubated with Fluos-IAPP. The inventors identified 3 major binding clusters corresponding to αSyn(1-13), αSyn(34-46), and αSyn(87-104) and a weaker one within the NAC region corresponding to αSyn(68-80) (Fig. 5e). Importantly, the 3 major IAPP-binding αSyn regions were nearly identical to the MCIP-binding ones which was consistent with the inventors' hypothesis. Furthermore, fluorescence spectroscopic titrations confirmed that the 3 major MCIP-binding αSyn segments bind (f)IAPP as well and revealed that the affinities of their interactions with IAPP were very similar to the affinities of their interactions with **2b** and **2e** (Table 2). Together, the above studies identified segments αSyn(1-14), αSyn(34-52), and αSyn(87-105) as key sites of the high affinity interactions of αSyn with both the MCIPs and IAPP.

The present inventors' findings suggest that MCIPs' multi-site binding to αSyn blocks interactions underlying αSyn misfolding cytotoxic di-/oligomerization, (self-)seeding, and fIAPP-mediated mediated cross-seeding and support the suggestion that multi-site targeting of αSyn could be a key requirement for effective anti-amyloid function. The present inventors' results also suggest that MCIPs' ability to mimic IAPP sites mediating IAPP/αSyn cross-interactions accounts for multi-site targeting of αSyn and support the notion that common molecular recognition features of Aβ, IAPP, and αSyn exist which can be exploited to develop multi-functional anti-amyloid molecules.

### Conclusion

The present inventors surprisingly show that macrocyclic peptides of formulae 0, 0a, 1, 1*, 2a - 2e, 2a* - 2e*, designed to mimic IAPP self-/cross-interaction sites and previously found to be potent inhibitors of amyloid self-assembly of IAPP and/or the amyloid-β peptide (Aβ) of Alzheimer's disease (AD), are also nanomolar inhibitors of both self- and IAPP-cross-seeded amyloid self-assembly of αSyn, as exemplified by, in particular, peptides 2b and 2e. The inventors' results suggest that their anti-amyloid function is mediated by nanomolar affinity interactions with αSyn *via* three αSyn segments which are identified as key sites of both αSyn self- and its cross-interactions with IAPP. Based on their broad spectrum amyloid inhibitor activity and additional drug-like properties, these macrocyclic peptides are now promising leads for multifunctional anti-amyloid drugs in synucleinopathies, such as PD and PD-related synucleinopathies, as well as T2D, AD, and their comorbidities. In addition, the identified key αSyn segments shall serve as valuable targets for the design of further novel, multi-site targeting molecules as effective anti-amyloids in PD, related synucleinopathies and their comorbidities.

### Claims

1. A peptide having an amino acid sequence according to formula 0

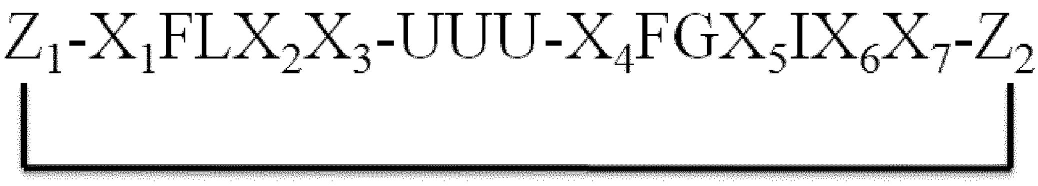

(Formula 0)

wherein

Z1 and Z2 are selected from the following pairs

a) cysteine and cysteine,
b) aspartic acid and lysine, or lysine and aspartic acid,
c) aspartic acid and ornithine, or ornithine and aspartic acid,
d) aspartic acid and 2,4-diaminobutyric acid, or 2,4-diaminobutyric acid and aspartic acid,
e) aspartic acid and 2,3-diaminopropionic acid, or 2,3-diaminopropionic acid and aspartic acid,
f) glutamic acid and lysine, or lysine and glutamic acid,
g) glutamic acid and ornithine, or ornithine and glutamic acid,
h) glutamic acid and 2,4-diaminobutyric acid, or 2,4-diaminobutyric acid and glutamic acid,
i) glutamic acid and 2,3-diaminopropionic acid, or 2,3-diaminopropionic acid and glutamic acid;

with 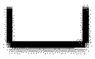 denoting a covalent bond between Z1 and Z2, thus providing for a cyclization of the peptide;
X1, X2, X3, X4, X5, X6, and X7 are, independently at each occurrence, selected from glycine, asparagine, valine, histidine, leucine, serine, alanine, and threonine;
F is, independently at each occurrence, phenylalanine;
L is leucine;
U is, independently at each occurrence, selected from arginine, homoarginine, citrulline, ornithine, lysine, and norleucine;
G is glycine;
I is isoleucine;
wherein Z1, Z2, X1-X7, F, L, U, G and I are L-amino acid residues or D-amino acid residues, or some of Z1, Z2, X1-X7, F, L, U, G and I are L-amino acid residues and others are D-amino acid residues;
and pharmaceutically acceptable salts, esters, solvates, polymorphs and modified forms thereof;
wherein preferably said peptide has an amino acid sequence according to formula 0a

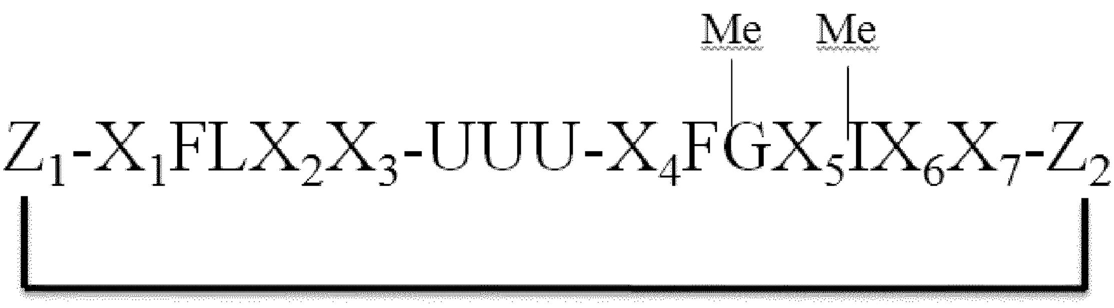

(Formula 0a)

wherein
Z1, Z2, X1-X7, F, L, U, G, I are as defined above, and

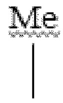

is N-methyl, for use in a method of treating, or preventing, or delaying the onset and/or pathogenesis of, a synucleinopathy.

**2.** The peptide for use according to claim 1, having an amino acid sequence according to formula 1

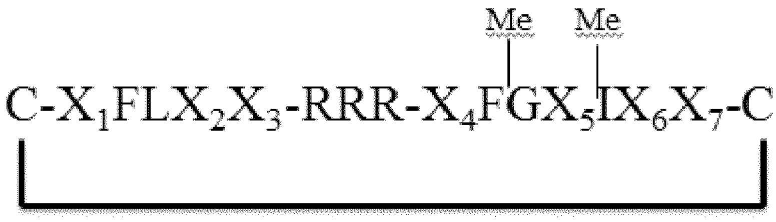

(Formula 1)

or an amino acid sequence according to formula 1*

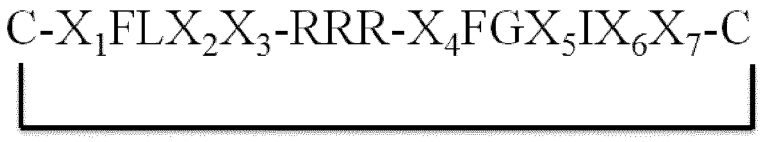

(Formula 1*)

wherein

C is cysteine;
X1, X2, X3, X4, X5, X6, and X7 are, independently at each occurrence, selected from glycine, asparagine, valine, histidine, leucine, serine, alanine, and threonine;
F is, independently at each occurrence, phenylalanine;
L is leucine;
R is arginine;
G is glycine;
I is isoleucine;

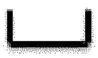 is a disulfide bond;

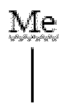

is N-methyl;

wherein C, X1-X7, F, L, R, G and I are L-amino acid residues or D-amino acid residues, or some of C, X1-X7, F, L, R, G and I are L-amino acid residues and others are D-amino acid residues;
and pharmaceutically acceptable salts, esters, solvates, polymorphs and modified forms thereof.

**3.** The peptide for use according to any of claims 1 and 2, wherein either

a) X1 and X4 are asparagine, X2 is valine, X3 is histidine, X4 is glycine, X5 is glycine, X6 and X7 are glycine;
b) X1-X7 are glycine, alanine or serine;
c) X1-X7 are glycine;
d) X1-X3 are glycine, X4 is asparagine, X5 is alanine, X6-X7 are glycine; or
e) X1-X3 are glycine, X4 is asparagine, X5 is alanine, X6 is leucine, X7 is serine.

**4.** The peptide for use according to any of claims 1 and 2, wherein Z1, Z2, C, X1-X7, F, L, U, R, G, and I are L-amino acid residues.

**5.** The peptide for use according to any of claims 1 - 3, wherein R is, at each occurrence, D-arginine, and/or

wherein F is, at each occurrence, D-phenylalanine, and/or
wherein L is D-leucine, and/or
wherein Z1, Z2 and C are D-amino acid residues, and/or
wherein I is D-isoleucine or N-methyl-D-isoleucine.

**6.** The peptide for use according to any of claims 1 -2, having a sequence according to a formula selected from the following formulae 2a - 2e, 2a* - 2e*:

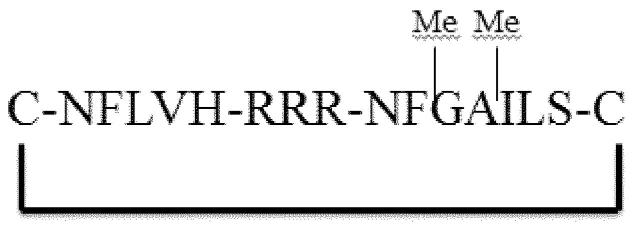

(Formula 2a)

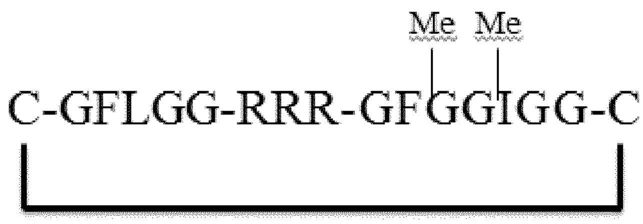

(Formula 2b)

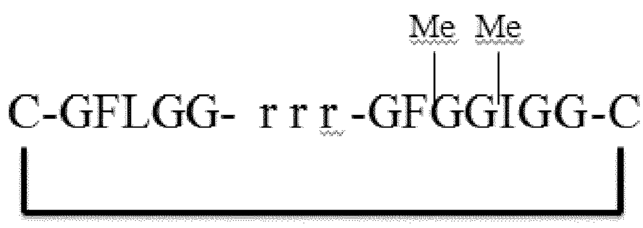

(Formula 2c)

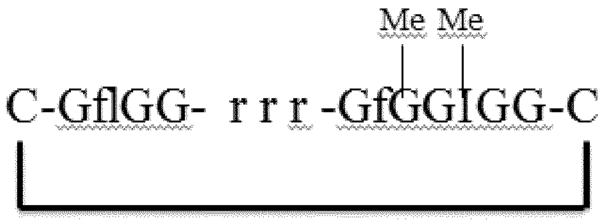

(Formula 2d)

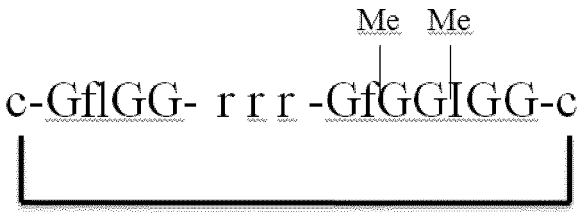

(Formula 2e)

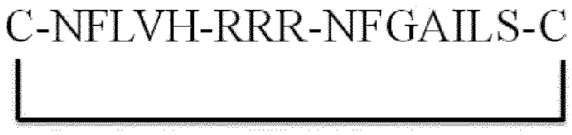

(Formula 2a*)

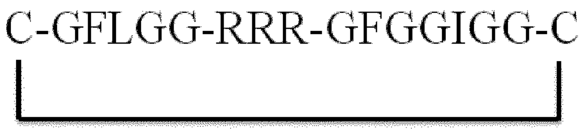

(Formula 2b*)

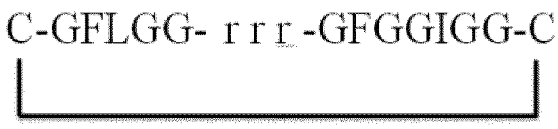

(Formula 2c*)

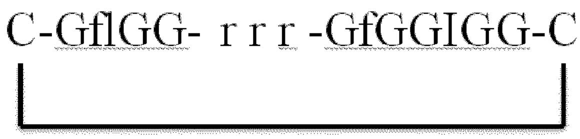

(Formula 2d*)

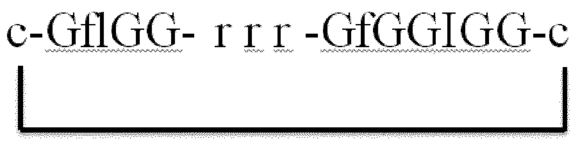

(Formula 2e*).

wherein upper case letters represent L-amino acid residues or D-amino acid residues, preferably L-amino acid residues, and lower case letters represent D-amino acid residues.

7. The peptide for use according to claim 6, having a sequence according to a formula selected from 2b, 2e, 2b* and 2e*

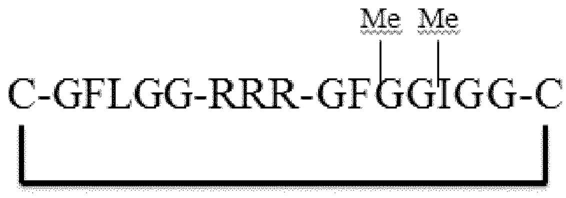

(Formula 2b)

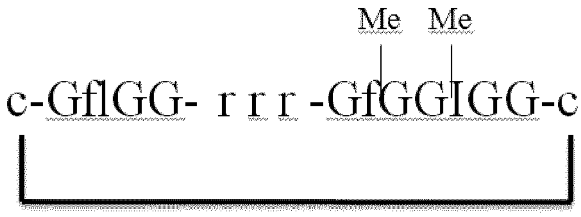

(Formula 2e)

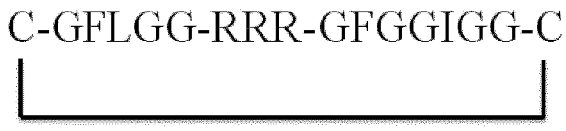

(Formula 2b*)

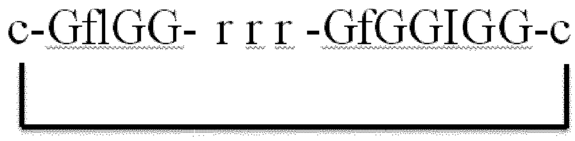

(Formula 2e*).

8. The peptide for use according to any of the foregoing claims, wherein said peptide consists of a sequence according to any of formulae 0, 0a, 1, 1*, 2a - 2e, 2a* - 2e*, as defined in any of claims 1, 2,6, and 7, respectively, preferably of a sequence according to any of formulae 2b, 2e, 2b* and 2e*, as defined in any of claims 1, 2, 6 and 7.

9. The peptide for us according to any of the foregoing claims, wherein said synucleinopathy is selected from Parkinson's disease (PD), dementia with Lewy bodies, multiple system atrophy, mitochondrial membrane protein associated neurodegeneration, and synucleinopathy-related comorbidities, including Parkinson's disease (PD)/Alzheimer's disease (AD), and Parkinson's disease (PD)/type 2 diabetes (T2D), wherein preferably, said synucleinopathy is Parkinson's disease (PD).

10. The peptide for use according to any of the foregoing claims, wherein said peptide is an amyloid inhibitory peptide that binds to α-synuclein (αSyn), preferably to monomers and/or oligomers and/or fibrils thereof, more preferably with nanomolar affinity.

11. A pharmaceutical composition comprising a peptide according to any of the foregoing claims and a pharmaceutically acceptable excipient, for use in a method of treating, or preventing, or delaying the onset and/or pathogenesis of, a synucleinopathy selected from Parkinson's disease (PD), dementia with Lewy bodies, multiple system atrophy, mitochondrial membrane protein associated neurodegeneration, and synucleinopathy-related comorbidities, including Parkinson's disease (PD)/Alzheimer's disease (AD), and Parkinson's disease (PD)/type 2 diabetes (T2D), wherein preferably, said synucleinopathy is Parkinson's disease (PD).

**12.** The peptide for use according to any of claims 1 - 10 or the pharmaceutical composition for use according to claim 11, wherein said method comprises administering an effective amount of said peptide or of said composition to a patient in need thereof.

**13.** The peptide as defined in any of claims 1 - 10 or the pharmaceutical composition as defined in claim 11, for use in a method of diagnosing a synucleinopathy which is selected from Parkinson's disease (PD), dementia with Lewy bodies, multiple system atrophy, mitochondrial membrane protein associated neurodegeneration, and synucleinopathy-related comorbidities, including Parkinson's disease (PD)/Alzheimer's disease (AD), and Parkinson's disease (PD)/type 2 diabetes (T2D), wherein preferably, said synucleinopathy is Parkinson's disease (PD).

**14.** The peptide or pharmaceutical composition for use according to claim 13, wherein said method comprises administering an effective amount of said peptide or of said composition to a subject to be tested for a synucleinopathy.

**15.** The peptide or pharmaceutical composition for use according to any of claims 13 - 14, wherein said peptide is linked to or administered together with a suitable reporter molecule that allows detection of α-synuclein (αSyn) and/or amyloid and/or non-amyloid aggregates and/or co-aggregates (e.g. aSyn/IAPP or aSyn/Abeta) and/or fibrils thereof by a suitable detection methodology, such as positron emission tomography (PET), nuclear magnetic resonance (NMR), magnetic resonance imaging (MRI), and PET-MRI and wherein said subject, after administration of said peptide, is subjected to said suitable detection methodology, such as PET, NMR, MRI, PET-MRI.

**16.** A kit for the in-vitro or in-vivo detection and/or quantification of α-synuclein (αSyn) and/or amyloid and/or non-amyloid aggregates and/or co-aggregates (e.g. aSyn/IAPP or aSyn/Abeta) and/or fibrils thereof, or for the diagnosis of a synucleinopathy selected from Parkinson's disease (PD), dementia with Lewy bodies, multiple system atrophy and mitochondrial membrane protein associated neurodegeneration, wherein preferably, said synucleinopathy is Parkinson's disease (PD), in a patient, said kit comprising the peptide as defined in any of claims 1 - 10, in a freeze-dried form in a suitable container, a buffered solvent in a separate container for reconstitution of said peptide in solution, and, optionally, means to dispense said peptide once reconstituted in solution, such as a syringe or pipette.

**17.** Use of the peptide as defined in any of claims 1 - 10, in an in-vitro assay, such as an enzyme linked immunosorbent assay (ELISA) or a radioimmuno assay (RIA), for the detection of α-synuclein (αSyn) monomeric or oligomeric aggregates and/or amyloid and/or non-amyloid aggregates and/or co-aggregates (e.g. aSyn/IAPP or aSyn/Abeta) and/or fibrils thereof.

Figure 1

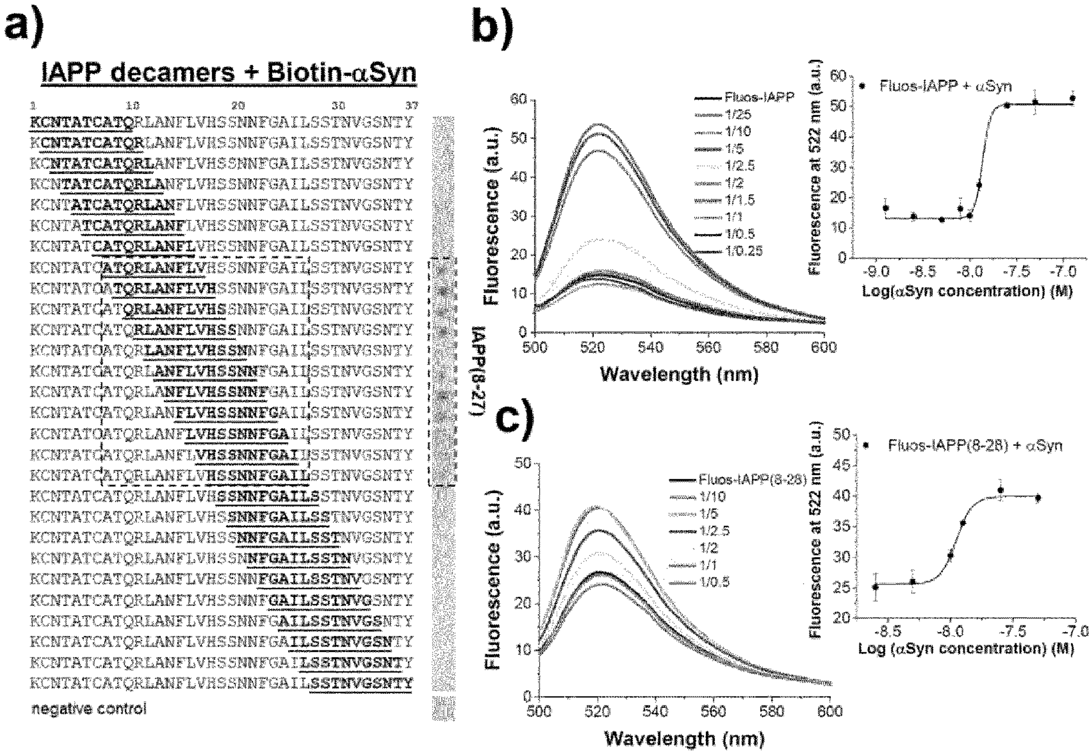
a)
IAPP decamers + Biotin-αSyn
1
10
20
30
37
KCNTATCATQRLANFLVHSSNNFGAILSSTNVGSNTY
negative control
IAPP(8-27)
b)
Fluos-IAPP
1/25
1/10
1/5
1/2.5
1/2
1/1.5
1/1
1/0.5
1/0.25
Fluorescence (a.u.)
Wavelength (nm)
Fluos-IAPP + αSyn
Fluorescence at 522 nm (a.u.)
Log(αSyn concentration) (M)
c)
Fluos-IAPP(8-28)
1/10
1/5
1/2.5
1/2
1/1
1/0.5
Fluorescence (a.u.)
Wavelength (nm)
Fluos-IAPP(8-28) + αSyn
Fluorescence at 522 nm (a.u.)
Log (αSyn concentration) (M)

Figure 2 a) – c)

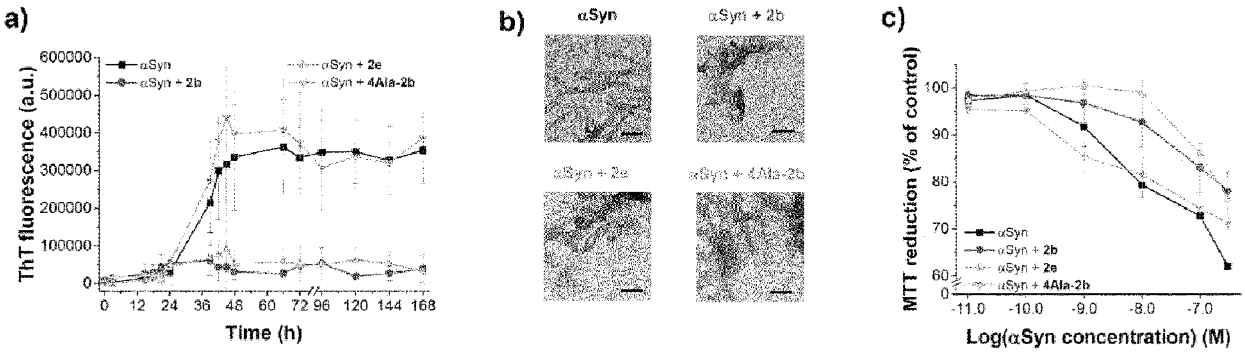
a)
ThT fluorescence (a.u.)
Time (h)
αSyn
αSyn + 2b
αSyn + 2e
αSyn + 4Ala-2b
b)
αSyn
αSyn + 2b
αSyn + 2e
αSyn + 4Ala-2b
c)
MTT reduction (% of control)
Log(αSyn concentration) (M)

Figure 2 d) – f)

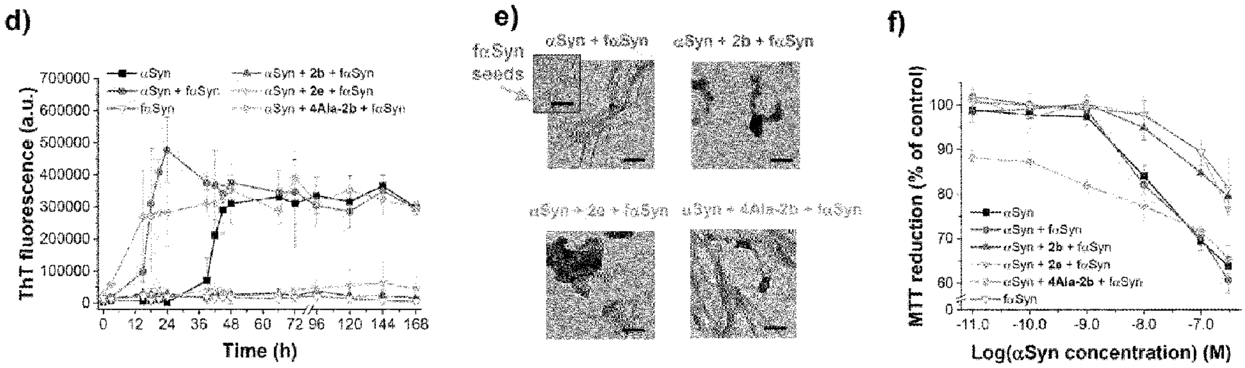
d)
e)
f)
αSyn + fαSyn
αSyn + 2b + fαSyn
fαSyn seeds
αSyn + 2e + fαSyn
αSyn + 4Ala-2b + fαSyn
ThT fluorescence (a.u.)
Time (h)
MTT reduction (% of control)
Log(αSyn concentration) (M)

Figure 2 g) – i)

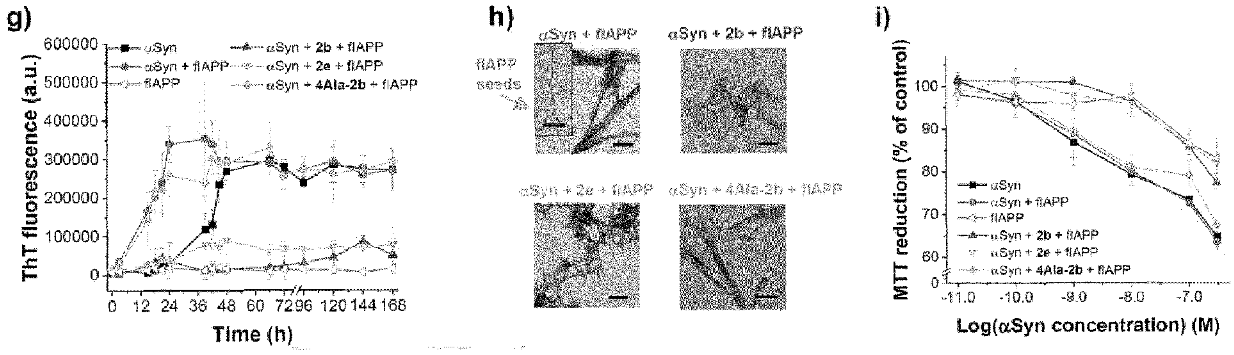
g)
h)
i)
αSyn + flAPP
αSyn + 2b + flAPP
αSyn + 2e + flAPP
αSyn + 4Ala-2b + flAPP
flAPP seeds
ThT fluorescence (a.u.)
Time (h)
MTT reduction (% of control)
Log(αSyn concentration) (M)

Figure 3 a) – b)

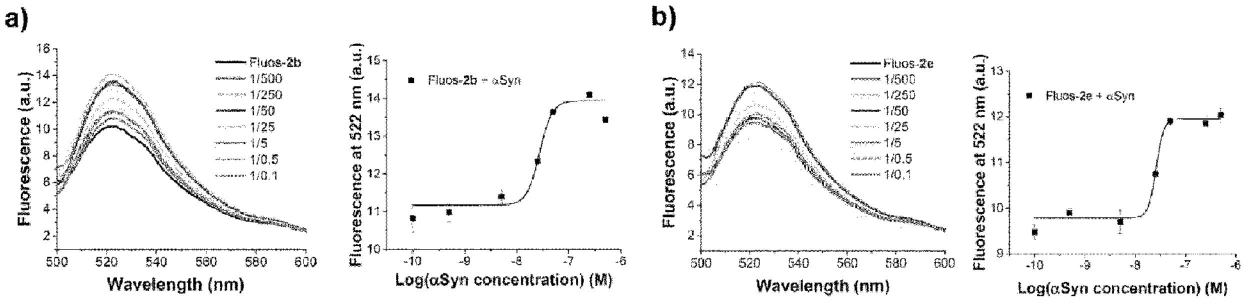
a)
Fluos-2b
1/500
1/250
1/50
1/25
1/5
1/0.5
1/0.1
Fluorescence (a.u.)
Wavelength (nm)
Fluos-2b + αSyn
Fluorescence at 522 nm (a.u.)
Log(αSyn concentration) (M)
b)
Fluos-2e
1/500
1/250
1/50
1/25
1/5
1/0.5
1/0.1
Fluorescence (a.u.)
Wavelength (nm)
Fluos-2e + αSyn
Fluorescence at 522 nm (a.u.)
Log(αSyn concentration) (M)

Figure 3 c) – e)

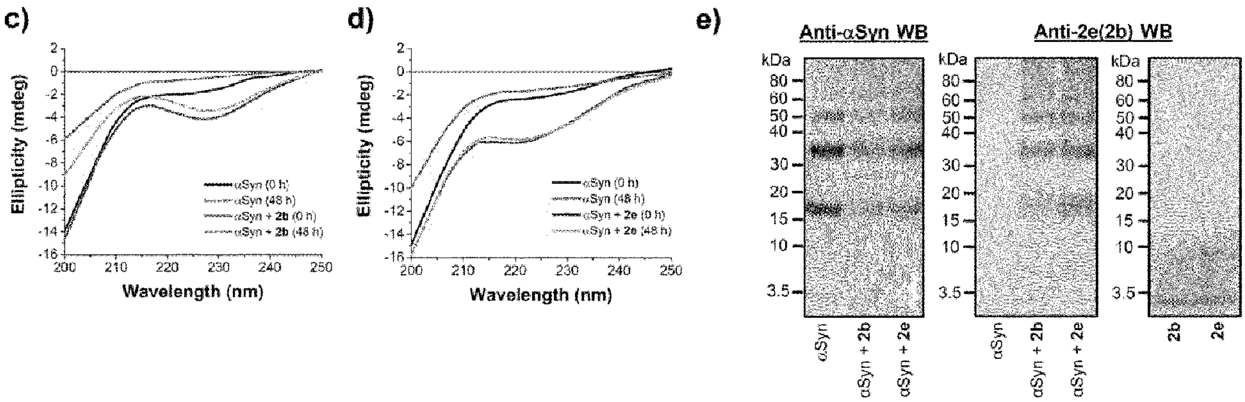
c)
Ellipticity (mdeg)
Wavelength (nm)
αSyn (0 h)
αSyn (48 h)
αSyn + 2b (0 h)
αSyn + 2b (48 h)
d)
Ellipticity (mdeg)
Wavelength (nm)
αSyn (0 h)
αSyn (48 h)
αSyn + 2e (0 h)
αSyn + 2e (48 h)
e)
Anti-αSyn WB
Anti-2e(2b) WB
kDa
αSyn
αSyn + 2b
αSyn + 2e
2b
2e

Figure 3 f) – h)

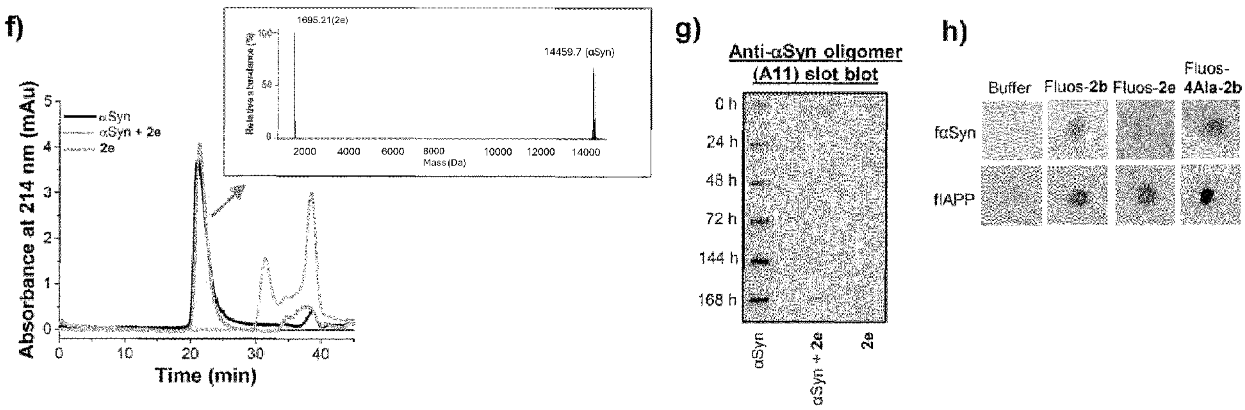
f)
1695.21(2e)
14459.7 (αSyn)
Relative abundance (%)
Mass (Da)
αSyn
αSyn + 2e
2e
Absorbance at 214 nm (mAu)
Time (min)
g)
Anti-αSyn oligomer (A11) slot blot
0 h
24 h
48 h
72 h
144 h
168 h
αSyn
αSyn + 2e
2e
h)
Buffer
Fluos-2b
Fluos-2e
Fluos-4Ala-2b
fαSyn
fIAPP

Figure 4

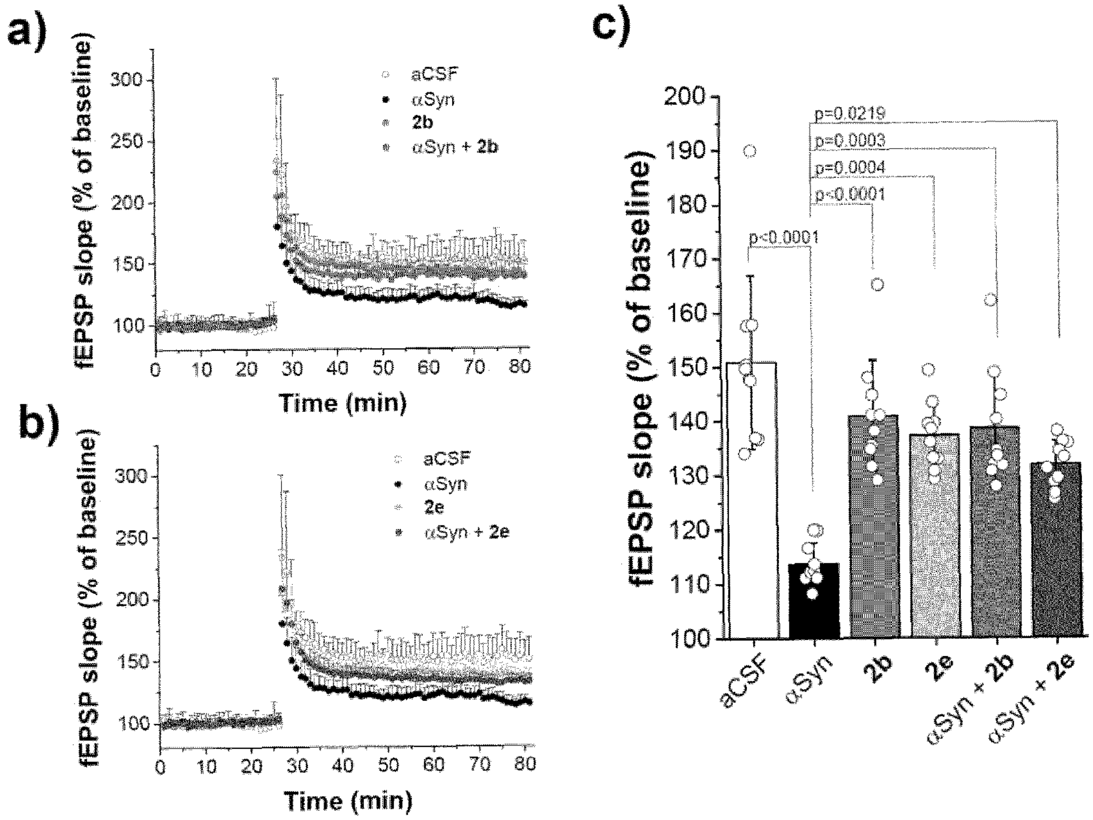
a)
fEPSP slope (% of baseline)
Time (min)
aCSF
αSyn
2b
αSyn + 2b
b)
fEPSP slope (% of baseline)
Time (min)
aCSF
αSyn
2e
αSyn + 2e
c)
fEPSP slope (% of baseline)
p=0.0219
p=0.0003
p=0.0004
p<0.0001
p<0.0001
aCSF
αSyn
2b
2e
αSyn + 2b
αSyn + 2e

Figure 5a)

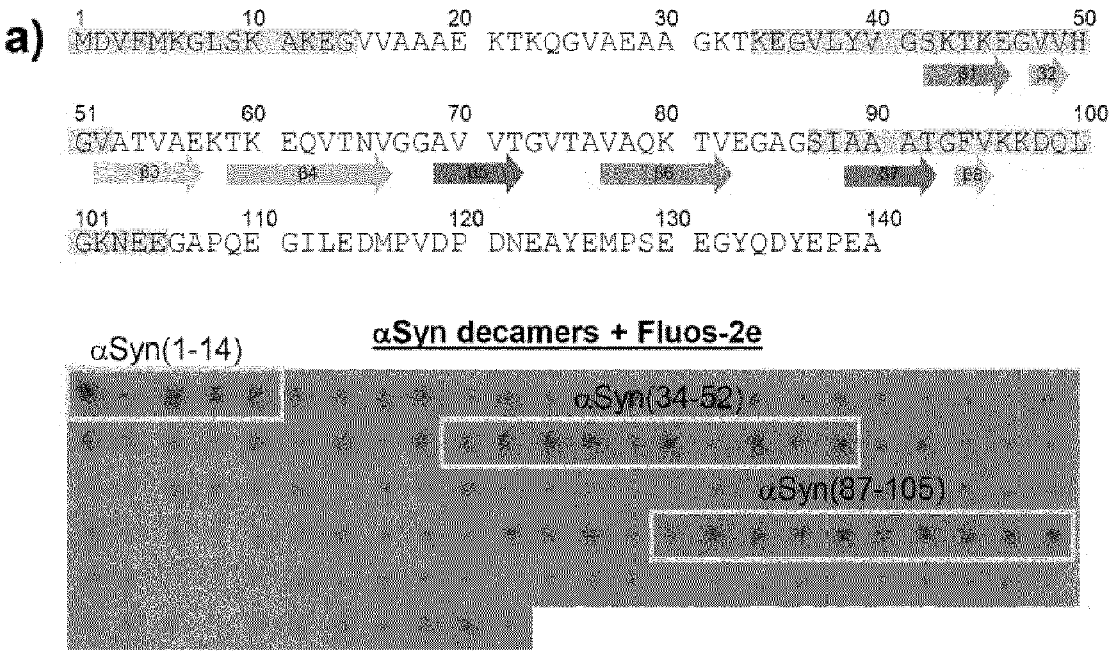
a)
1 10 20 30 40 50
MDVFMKGLSK AKEGVVAAAE KTKQGVAEAA GKTKEGVLYV GSKTKEGVVH
51 60 70 80 90 100
GVATVAEKTK EQVTNVGGAV VTGVTAVAQK TVEGAGSIAA ATGFVKKDQL
101 110 120 130 140
GKNEEGAPQE GILEDMPVDP DNEAYEMPSE EGYQDYEPEA
αSyn decamers + Fluos-2e
αSyn(1-14)
αSyn(34-52)
αSyn(87-105)

Figure 5 b) – d)

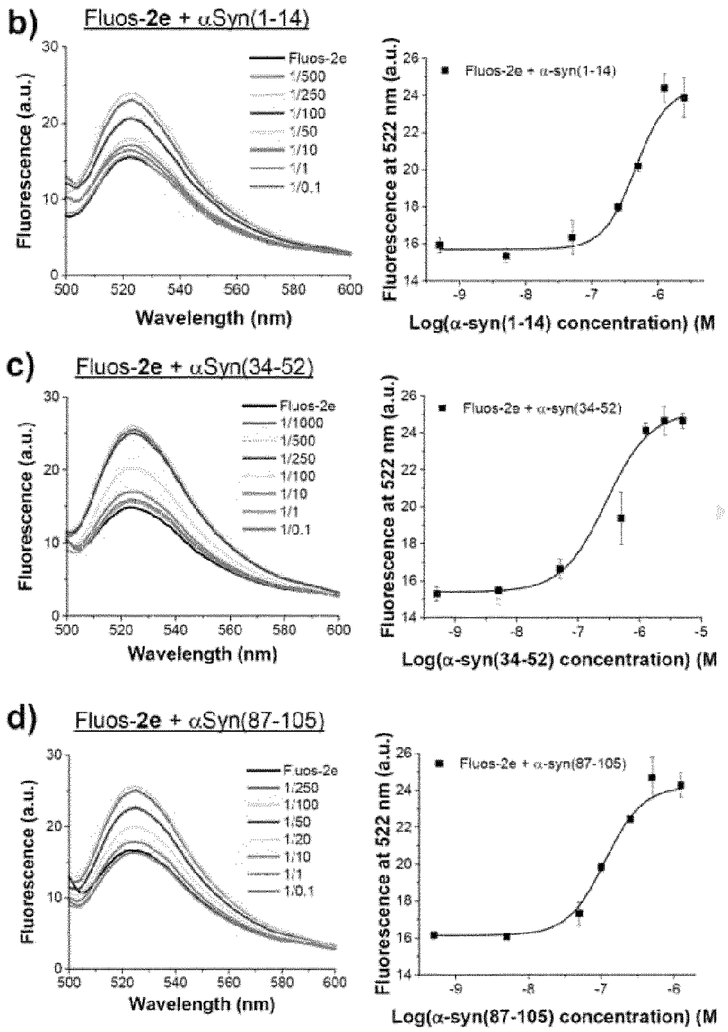
b)
Fluos-2e + αSyn(1-14)
Fluorescence (a.u.)
Wavelength (nm)
Fluos-2e
1/500
1/250
1/100
1/50
1/10
1/1
1/0.1
Fluorescence at 522 nm (a.u.)
Fluos-2e + α-syn(1-14)
Log(α-syn(1-14) concentration) (M
c)
Fluos-2e + αSyn(34-52)
Fluorescence (a.u.)
Wavelength (nm)
Fluos-2e
1/1000
1/500
1/250
1/100
1/10
1/1
1/0.1
Fluorescence at 522 nm (a.u.)
Fluos-2e + α-syn(34-52)
Log(α-syn(34-52) concentration) (M
d)
Fluos-2e + αSyn(87-105)
Fluorescence (a.u.)
Wavelength (nm)
Fluos-2e
1/250
1/100
1/50
1/20
1/10
1/1
1/0.1
Fluorescence at 522 nm (a.u.)
Fluos-2e + α-syn(87-105)
Log(α-syn(87-105) concentration) (M

Figure 5e)

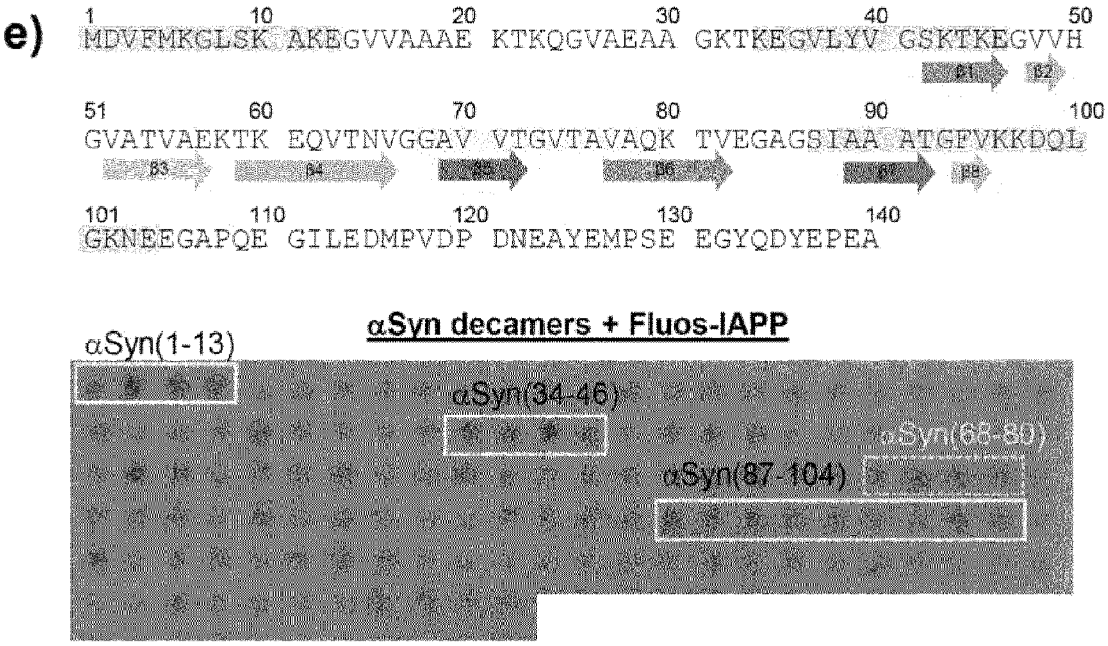
e)
1 10 20 30 40 50
MDVFMKGLSK AKEGVVAAAE KTKQGVAEAA GKTKEGVLYV GSKTKEGVVH
51 60 70 80 90 100
GVATVAEKTK EQVTNVGGAV VTGVTAVAQK TVEGAGSIAA ATGFVKKDQL
101 110 120 130 140
GKNEEGAPQE GILEDMPVDP DNEAYEMPSE EGYQDYEPEA
αSyn decamers + Fluos-IAPP
αSyn(1-13)
αSyn(34-46)
αSyn(68-80)
αSyn(87-104)

Figure 5f)

f)

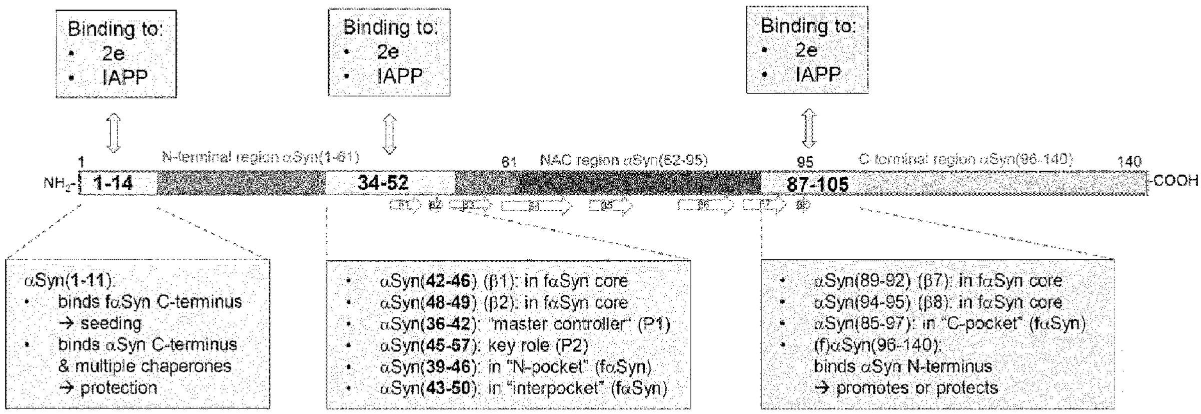
Binding to:
• 2e
• IAPP
Binding to:
• 2e
• IAPP
Binding to:
• 2e
• IAPP
N-terminal region αSyn(1-61)
NAC region αSyn(62-95)
C terminal region αSyn(96-140)
1
61
95
140
NH2-
1-14
34-52
87-105
-COOH
αSyn(1-11):
• binds fαSyn C-terminus → seeding
• binds αSyn C-terminus & multiple chaperones → protection
• αSyn(42-46) (β1): in fαSyn core
• αSyn(48-49) (β2): in fαSyn core
• αSyn(36-42): "master controller" (P1)
• αSyn(45-57): key role (P2)
• αSyn(39-46): in "N-pocket" (fαSyn)
• αSyn(43-50): in "interpocket" (fαSyn)
• αSyn(89-92) (β7): in fαSyn core
• αSyn(94-95) (β8): in fαSyn core
• αSyn(85-97): in "C-pocket" (fαSyn)
• (f)αSyn(96-140): binds αSyn N-terminus → promotes or protects

Figure 6

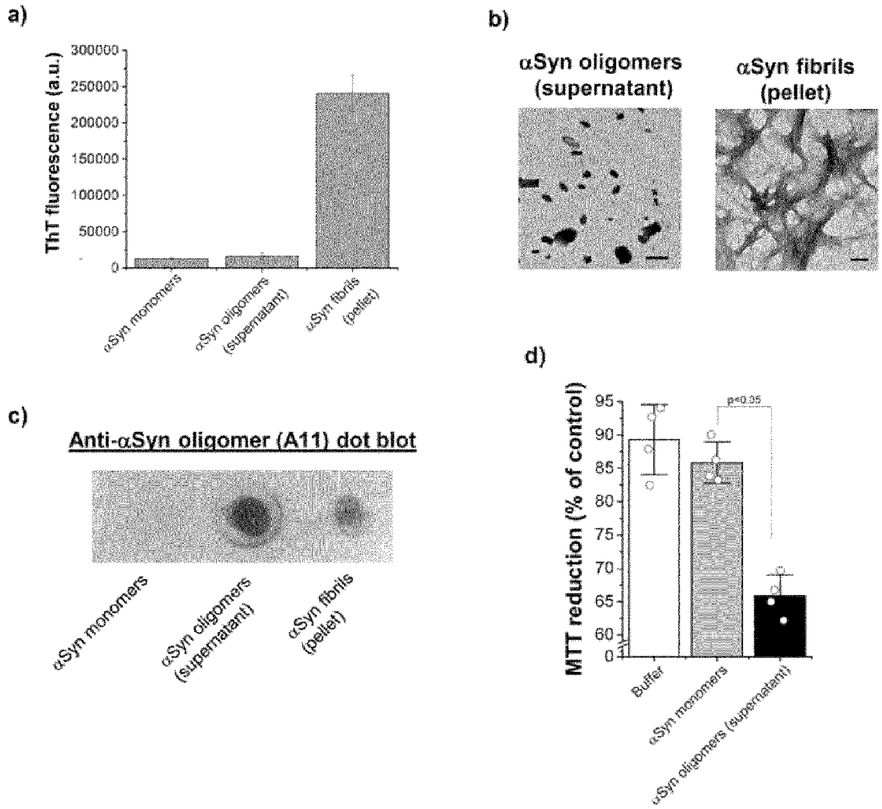
a)
ThT fluorescence (a.u.)
300000
250000
200000
150000
100000
50000
0
αSyn monomers
αSyn oligomers (supernatant)
αSyn fibrils (pellet)
b)
αSyn oligomers (supernatant)
αSyn fibrils (pellet)
c)
Anti-αSyn oligomer (A11) dot blot
αSyn monomers
αSyn oligomers (supernatant)
αSyn fibrils (pellet)
d)
MTT reduction (% of control)
95
90
85
80
75
70
65
60
0
p<0.05
Buffer
αSyn monomers
αSyn oligomers (supernatant)

Europäisches Patentamt
European Patent Office
Office européen des brevets

# EUROPEAN SEARCH REPORT

Application Number
EP 24 19 2715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/234157 A1 (UNIV MUENCHEN TECH [DE]) 12 December 2019 (2019-12-12)<br>* examples, figures, claims *<br>----- | 1-17 | INV.<br>C07K7/08<br>G01N33/68 |
| X | ANNA SPANOPOULOU ET AL: "Designed Macrocyclic Peptides as Nanomolar Amyloid Inhibitors Based on Minimal Recognition Elements",<br>ANGEWANDTE CHEMIE INTERNATIONAL EDITION, VERLAG CHEMIE, HOBOKEN, USA,<br>vol. 57, no. 44, 24 July 2018 (2018-07-24), pages 14503-14508, XP072102861,<br>ISSN: 1433-7851, DOI: 10.1002/ANIE.201802979<br>* the whole document *<br>----- | 1-17 | |
| A | IKENOUE TATSUYA ET AL: "A RaPID Macrocyclic Peptide That Inhibits the Formation of [alpha]-Synuclein Amyloid Fibrils",<br>CHEMBIOCHEM,<br>vol. 24, no. 12, 15 June 2023 (2023-06-15), XP093233355,<br>Hoboken, USA<br>ISSN: 1439-4227, DOI: 10.1002/cbic.202300320<br>Retrieved from the Internet:<br>URL:https://chemistry-europe.onlinelibrary.wiley.com/doi/pdfdirect/10.1002/cbic.202300320><br>----- | 1-15 | TECHNICAL FIELDS SEARCHED (IPC)<br>C07K<br>G01N |

The present search report has been drawn up for all claims

1

| Place of search | Date of completion of the search | Examiner |
|---|---|---|
| Munich | 20 January 2025 | Behrens, Ralf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 19 2715

This annex lists the patent family members relating to the patent documents cited in the above-mentioned European search report. The members are as contained in the European Patent Office EDP file on
The European Patent Office is in no way liable for these particulars which are merely given for the purpose of information.

20-01-2025

| Patent document cited in search report | Publication date | Patent family member(s) | Publication date |
|---|---|---|---|
| WO 2019234157 A1 | 12-12-2019 | EP 3578565 A1 | 11-12-2019 |
| | | EP 3774846 A1 | 17-02-2021 |
| | | US 2021340180 A1 | 04-11-2021 |
| | | US 2023095144 A1 | 30-03-2023 |
| | | WO 2019234157 A1 | 12-12-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7745490 B
- WO 2019234157 A
- WO 2019234157 A1


### Non-patent literature cited in the description


- **S. T. KUMAR** ; **S. DONZELLI** ; **A. CHIKI** ; **M. M. K. SYED** ; **H. A. LASHUEL**. *Journal of Neurochemistry*, 2020, vol. 153, 103-119
- **K. TAS** ; **B. D. VOLTA** ; **C. LINDNER** ; **O. EL BOUNKARI** ; **K. HILLE** ; **Y. TIAN** ; **X. PUIG-BOSCH** ; **M. BALLMANN** ; **S. HORNUNG** ; **M. ORTNER**. *Nat Commun*, 2022, vol. 13, 5004
- **SHAYKHALISHAHI, A. GAUHAR** ; **M. M. WORDEHOFF** ; **C. S. GRUNING** ; **A. N. KLEIN** ; **O. BANNACH** ; **M. STOLDT** ; **D. WILLBOLD** ; **T. HARD** ; **W. HOYER**. *Angew Chem Int Ed Engl*, 2015, vol. 54, 8837-8840
- **M. WÖRDEHOFF** ; **W. HOYER**. *Bio-protocol*, 2018, 8
- **M. YAN** ; **A. VELKOVA** ; **M. TATAREK-NOSSOL** ; **E. ANDREETTO** ; **A. KAPURNIOTU**. *Angew Chem Int Ed Engl*, 2007, vol. 46, 1246-1252
- **A. SPANOPOULOU** ; **L. HEIDRICH** ; **H. R. CHEN** ; **C. FROST** ; **D. HRLE** ; **E. MALIDELI** ; **K. HILLE** ; **A. GRAMMATIKOPOULOS** ; **J. BERNHAGEN** ; **M. ZACHARIAS**. *Angew Chem Int Ed Engl*, 2018, vol. 57, 14503-14508
- **ANDREETTO, E. MALIDELI** ; **L. M. YAN** ; **M. KRACKLAUER** ; **K. FARBIARZ** ; **M. TATAREK-NOSSOL** ; **G. RAMMES** ; **E. PRADE** ; **T. NEUMULLER** ; **A. CAPORALE** ; **A. SPANOPOULOU**. *Angew Chem Int Ed Engl*, 2015, vol. 54, 13095-13100
- **TAS, B. D. VOLTA** ; **C. LINDNER** ; **O. EL BOUNKARI** ; **K. HILLE** ; **Y. TIAN** ; **X. PUIG-BOSCH** ; **M. BALLMANN** ; **S. HORNUNG** ; **M. ORTNER** ; **S. PREM**. *Nat Commun*, 2022, vol. 13, 5004
- **Z. ZHANG** ; **A. G. MARSHALL**. *Journal of the American Society for Mass Spectrometry*, 1998, vol. 9, 225-233
- **C. KONTOS** ; **O. EL BOUNKARI** ; **C. KRAMMER** ; **D. SINITSKI** ; **K. HILLE** ; **C. ZAN** ; **G. YAN** ; **S. WANG** ; **Y. GAO** ; **M. BRANDHOFER**. *Nat Commun*, 2020, vol. 11, 5981
- **ANDREETTO, L. M. YAN** ; **M. TATAREK-NOSSOL** ; **A. VELKOVA** ; **R. FRANK** ; **A. KAPURNIOTU**. *Angew Chem Int Ed Engl*, 2010, vol. 49, 3081-3085
- **M. J. DIOGENES** ; **R. B. DIAS** ; **D. M. ROMBO** ; **H. VICENTE MIRANDA** ; **F. MAIOLINO** ; **P. GUERREIRO** ; **T. NASSTROM** ; **H. G. FRANQUELIM** ; **L. M. OLIVEIRA** ; **M. A. CASTANHO**. *Journal of Neuroscience*, 2012, vol. 32, 11750-11762
- **K. J. ROTHMAN**. *Epidemiology*, 1990, vol. 1, 43-46
- **ANDREETTO, KAPURNIOTU et al.** *ACIE*, 2015
- **SPANOPOULOU, KAPURNIOTU et al.** *ACIE*, 2018